# EUROPEAN PATENT APPLICATION

(11) **EP 1 990 765 A1**
(43) Date of publication of application: **12.11.2008**
(21) Application number: 07708043.0
(22) Date of filing: 05.02.2007
(51) Int. Cl.: G06Q 50/00

(54) **MEDICAL IMAGE SYSTEM**

(30) Priority: 02.03.2006 JP 2006056448; 16.06.2006 JP 2006167297
(71) Applicant: Konica Minolta Medical & Graphic, Inc., Hino-shi, Tokyo 191-8511 (JP)
(72) Inventor: SHIIBASHI, Takao, Shinjuku-ku, Tokyo 1630512 (JP)
(74) Representative: Gille Hrabal Struck Neidlein Prop Roos
(86) International application number: PCT/JP2007/051925
(87) International publication number: WO 2007/099735

(57) **Abstract**

A medical imaging system by which in order that image data generated by an image generating apparatus and patient information are efficiently associated with each other while the doctor is examining the patient, an on-line button is blinking on receiving the image data or examination data when a screen showing information on the specified patient is displayed, the received data is automatically associated with the designated patient information and stored in an image DB when the blinking on-line button is depressed, and the received data is temporarily stored in a temporary storage section for storing data waiting for association with the patient information when the blinking on-line button is held depressed long.

## Description

### FIELD OF THE INVENTION

The present invention relates to a medical imaging system used mainly in small-sized medical facilities, particularly to a medical imaging system capable of displaying a list of a captured image of the examined region of a patient, examination information and/or treatment information.

### BACKGROUND OF ART

One of the techniques known in the conventional art is a diagnostic system wherein a radiographing technician takes a radiograph of the outpatient as an object of examination using an image generating apparatus such as a CR (Computed Radiography) apparatus or an FPD (Flat Panel Detector), and applies image processing such as gradation processing to ensure that the captured image can be used for diagnosis, whereby the processed image is outputted as a radiograph for interpretation by a doctor.

In such a diagnostic system, diagnosis is carried out by a plurality of persons in charge of different functions such as a person in charge of receiving an outpatient and issuing a radiographing order (receptionist), a person in charge of radiographing the patient in an X-ray radiographing room and generating image data (radiographing technician), a person in charge of determining the suitability of the captured image for application to the diagnosis, for example, by studying gradability, and correcting the contrast and density in some cases (a radiographing technician appointed from among general radiographing technicians), and a person in charge of interpreting the radiograph to determine (diagnose) the presence or absence of a disease based on the image.

In large-sized medical facilities (hereinafter referred to as "large-sized facilities") wherein application of the conventional system is assumed, there are a plurality of image generating apparatuses and a plurality of radiographing technicians operating the image generating apparatuses. The consoles for operating the image generating apparatus and the viewers used by a doctor to check image data are assigned with respective roles and are separately installed. This may lead to confusion between the patients and image data. To prevent this confusion, a system has been proposed wherein various apparatuses are linked via the network. An ID is issued by each apparatus, and the results of the work process implemented in each apparatus are associated with one another (e.g., Patent Document 1).

In such a system, the places wherein the aforementioned roles are played are separated from one another in a large hospital in many cases. For example, a reception desk is located on the first floor, and the department of radiology is found in the basement. In the department of radiology, it is a common practice that radiographing operations for a plurality of patients are performed simultaneously by a plurality of radiographing technicians using a plurality of radiographing apparatuses. A plurality of patients are made to wait in each process at all times, and ID is assigned to each job in each process to ensure correct association between the generated images and individual patients. Association is provided via the HIS network (Hospital Information System) and RIS (Radiology Information System) (Patent Documents 2 and 3).

For example, the radiographing details are determined at the first floor reception desk based on the present complaint of a patient and are registered together with the name of the patient. This patient list is added whenever necessary and is displayed on the first floor reception workstation (hereinafter referred to as "WS"). At the same time, the patient list is displayed on the console (which is a workstation located in the department of radiology to display the radiographing condition setting, the radiographing order information of the RIS/HIS, and a radiographic image of the patient) in the radiology department in the basement via a network, such as RIS, HIS or others. It is a common practice to install a plurality of consoles to improve the efficiency of decentralized processing. They are connected among one another via the network. When a predetermined radiographing ID is selected on any console, duplicate radiography among a plurality of radiographing technicians is avoided by using a method that notifies in the list of a patient if the patient is being processed (by a flashing display, a change of color or a beep alarm when an identical examination is designated).

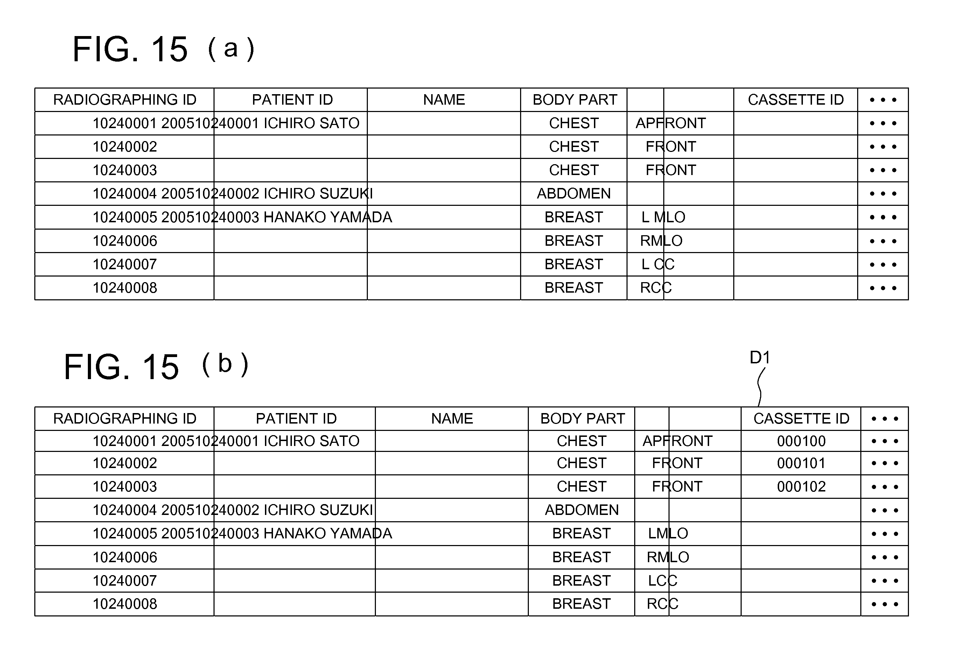

Using the console located at hand, the radiographing technician selects a radiographing ID for radiographing from the displayed list of a patient, and registers the ID (cassette ID) of the CR plate (cassette) to be used. This procedure allows the cassette ID registered to be displayed in the column of the cassette ID (Dl), as shown in Fig. 15(b). The radiographing technician picks up three cassettes, for example, and goes into the X-ray radiographing room to take a radiographic picture of the patient. Then the cassette containing the captured image is read by the cassette reader. The cassette reader reads out the cassette ID affixed to the inserted cassette. This is attached to the image data and is then transmitted. Finally, the radiographing ID (patient ID) is associated with the generated image data. The generated image data is sent to the console wherein the radiographing ID is selected by the radiographing technician, and is displayed on the console. In this phase, radiographic positioning is checked. If positioning is not satisfactory, the patient is radiographed again, and a decision is also made to see whether or not the density and contrast should be corrected, and whether or not the frequency enhancement processing should be performed. After that, this image is stored in a server to wait for radiographic interpretation (wait for diagnosis). Then the doctor in charge of radiographic interpretation operates the workstation (often provided with a high-definition monitor for viewer function) of an interpretation room so that the image of a particular patient is selected from the image stored in the server wherein data is waiting for radiographic interpretation, and is displayed on this workstation. Thus, radiographic interpretation is performed by the doctor.

The system used in such a large-sized facility registers the radiographing ID of the patient and cassette ID whereby association between the patient and radiographic image is provided. This arrangement ensures that all pieces of information including the information on calculation of medical insurance points, for example, on the type of radiographic operation such as whether the radiography applied to a patient is simple radiography or contrast radiography, is linked with one another so that concentrated control is provided at the RIS/HIS server.

However, according to the survey by the inventors of the present patent application, in comparatively small medical facilities such as medical practitioner's offices or clinics (hereinafter referred to as "small-sized facilities"), only one doctor takes charges of diagnosis of a patient in many cases, and only a small number of the image generating apparatuses are installed. An assistant takes charge of positioning the patient. Upon notification of the completion of positioning by the assistant, the doctor controls the X-ray switch in many cases. Furthermore, only one doctor often has to take care of positioning of the patient as well.

Further, in the case of large-sized facilities, it is considered that a patient has to move from floor to floor within the facility throughout the processes from radiographing to the diagnosis by the doctor. In the case of small-sized facilities, the patient moves only a small distance throughout the processes from radiographing to the diagnosis by the doctor, since the facilities are smaller.

Under such circumstances, it is not very likely that the radiographic image of a patient is taken for that of another patient. Use of a system similar to that of a large-sized facility will lower diagnostic efficiency since it is necessary to perform the work for generating the radiographic order information including the input of the patient name, and a complicated procedure is needed.

To generate the radiographic order information such as the patient information and radiographing condition information in advance, and to associate this radiographic order information with the radiographic image having been captured, it is necessary to build a system for linking various apparatuses via the network associated with the basic system such as the RIS/HIS. However, this comes at a high cost. It is also necessary to configure a device to ensure the consistency of data with the basic system (e.g. Patent Documents 4 and 5). This represents a great burden to small-scale facilities. If the number of apparatuses are reduced using the same configuration concept of the aforementioned large-sized facilities, this cannot be directly applicable to small-sized facilities.

In the small-sized medical facilities such as medical practitioner's office or clinics (hereinafter referred to as "small-sized facilities"), there has been widespread use of electronic patient medical charts. This permits searching for descriptions in the patient medical charts. The operation of inputting the information in the electronic patient medical charts causes a heavier burden on the doctor than manually filling in the paper patient medical charts. Many methods have been proposed, as exemplified by the method of reducing the inputting load, as shown in Patent Document 6.

In the PACS (Picture Archiving Communication System:
Patent Document 7) and electronic patient medical chart system, the examination order input is used as a key, and the image information, report information, and treatment information generated thereafter are associated therewith. These pieces of information are displayed in a list to facilitate finding the clinical path, as disclosed in the Patent Document 8.
Patent Document 1: U.S. Patent No. 5,334,851: Specification
Patent Document 2: Japanese Unexamined Patent Application Publication No. 2002-159476
Patent Document 3: Japanese Unexamined Patent Application Publication No. 2002-311524
Patent Document 4: Japanese Unexamined Patent Application Publication No. 2006-92261
Patent Document 5: Japanese Unexamined Patent Application Publication No. 2003-248723
Patent Document 6: Japanese Unexamined Patent Application Publication No. 2002-7021
Patent Document 7: Japanese Unexamined Patent Application Publication No. 5-12352
Patent Document 8: Japanese Unexamined Patent Application Publication No. 2003-132144

### DISCLOSURE OF THE INVENTION

### PROBLEMS TO BE SOLVED BY THE INVENTION

An object of the present invention is to find out the way of efficiently associating the image data generated by an image generating apparatus and patient information when a doctor is examining a patient.

The aforementioned PACS and electronic patient medical chart system, require equipment investment for network linkage of various facilities in a hospital, and input operations for captured image interpretation reports using a keyboard. A large-sized hospital is staffed with expert members to take care of various roles. To put it another way, the system is implemented by human investment. It is difficult to simply apply such a method to the small-sized facilities of medical practitioners or the like wherein one doctor has to play the roles of many persons. In the conventional circle of medical practitioners, a predominantly great number of doctors use paper for the patient medical chart system. There is no implementation of a computerized system wherein examination order is issued for medical examination and image capturing operation for a patient, and this examination order is used as a key for association with the image data and report information (patient medical chart information) to be generated subsequently. Thus, the accompanying disadvantage is that an installation of a network must be provided for a list display, and there are an increased number of man hours for the new computer input operations.

In the small-sized facilities of a medical practitioner or the like, the doctor is often required to perform examinations by himself. There is almost no case of introducing the ordering apparatus which is usually introduced into a large-sized hospital for generating orders related to medical care (prescription, treatment, and examination), which is effective in the case of hospitals staffed with a great number of human resources. In such a system for medical practitioners, the efficiency of medical care greatly depends on how efficiently the summary of the overall treatment implemented by a doctor can be efficiently displayed on a list and can be referenced (with a paper patient medical chart at hand) after implementation, independently of the presence or absence of a treatment order. Especially the captured image of the patient is interpreted using a film in a predominant number of cases. It will be difficult to secure the association between the patient and image at the time of film-less work anticipated in future (at the time of image diagnosis by monitor).

To solve these problems, the present inventors made an in-hospital flow analysis for medical practitioner's office and clinics, and have found out that, even in the facilities wherein paper patient medical charts are used without the system being computerized, an expert staff member other than the doctor is assigned for reception and accounting work in many cases, and is engaged in inputting the reception-related information such as the image-captured region information, the number of shots and size of the radiograph, and accounting work for patients, using a personal computer (workstation), and new input work for displaying a list can be omitted through effective use of this input information.

A further object to be achieved by the present invention is to find out a way to ensure that the past diagnostic history is displayed in a list on the screen by a simple inputting operation in the facilities of the medical practitioners having the aforementioned problems. It is also necessary to ensure that the screen wherein the past medical care history is displayed in a list can be changed to the captured image data as a basis for diagnosis. A further requirement is to ensure that the aforementioned display in a list can also be applied to the film-less work, and to enhance usability by medical practitioners and in clinics.

### MEANS FOR SOLVING THE PROBLEMS

To solve the aforementioned problems, the present invention described in Claim 1 provides a medical imaging system wherein an image generating apparatus for capturing an image of the region of a patient to be examined and generating image data is linked with a medical image controlling apparatus that associates the image data generated by the aforementioned image generating apparatus with the patient information in such a way that data can be sent and received between them; wherein the aforementioned medical image controlling apparatus includes:
a patient list acquiring device for acquiring the patient information list of a patient to be examined;
a patient designating device for designating the patient information for the patient to be examined, from the acquired patient information list;
a control mode selecting device for selecting the control mode when the image data has been received from the image generating apparatus; and
a control device containing a first control mode wherein, when image data is received from the aforementioned image generating apparatus, the received image data is automatically associated with the patient information designated by the patient designating device, and is stored in a storage device; and a second control mode wherein the received image data, without being associated with the patient information, is stored in a temporary storing device; and the received image data is subjected to storage control in the first or second control mode based on the selection by the control mode selecting device.

The invention described in Claim 2 is based on that described in Claim 1 further characterized in that the aforementioned image generating apparatus is installed in an X-ray radiographing room and the medical image controlling apparatus is installed in a doctor consultation room.

The invention described in Claim 3 is based on that described in Claim 1 or 2 further characterized in that the aforementioned medical image controlling apparatus is provided with a notifying device for notifying of reception of the image data from the image generating apparatus.

The invention described in Claim 4 is based on that described in Claim 3 further characterized in that the aforementioned notifying device notifies of the reception every time the image data is received from the image generating apparatus.

The invention described in Claim 5 is based on that described in any one of the Claims 1 through 4 further characterized in that the medical image controlling apparatus is connected with a plurality of image generating apparatuses.

The invention described in Claim 6 is based on that described in Claim 5 further characterized in that the aforementioned control mode selecting device is capable of selecting the control mode in response to each of the aforementioned connected image generating apparatuses.

The invention described in Claim 7 is based on that described in any one of the Claims 1 through 6 further characterized in that:
the aforementioned medical image controlling apparatus further includes a third control mode wherein;
when image data is received from the image generating apparatus, the received image data is associated with the patient information different from that designated by the patient designating device; and wherein
when the third control mode is selected by the control mode selecting device, the aforementioned control device displays the patient information list acquired by the patient list acquiring device on the display device with patient information being selectable, and associates the patient information selected from the displayed patient information list with the received image data, whereby the received image data is stored in the storage device.

The invention described in Claim 8 is based on that described in any one of the Claims 1 through 7 further characterized in that:
the medical image controlling apparatus is linked with an examination apparatus for acquiring patient examination data in such a way that the data can be sent and received;
the control mode selecting device is capable of selecting the control mode when the examination data has been received from the examination apparatus;
the control device, when examination data has been received from the examination apparatus, automatically associates the received examination data with the patient information designated by the patient designating device based on the selection of the control mode selecting device, and stores the data in the examination information storing device; or stores the received examination data into the temporary storing device, without the examination data being associated with the patient information.

To solve the aforementioned problems, the invention described in Claim 9 is based on that described in any one of the Claims 1 through 7 further including:
a reception device for registering the information for separating the patients individually and information on reception date for reception of the patient;
a patient information input device for inputting the patient information by associating the patient information with the information for separating the patients individually and information on reception date having been registered by the aforementioned reception device;
an image storing device for storing the image data generated by the image generating apparatus, the patient information on the patient whose image to be captured, and the information on the date of capturing the image data, which have been associated to one another;
a reception-related information input device for inputting reception-related information including the computerized examination information and/or the treatment information corresponding to the information for separating the patients individually and information on reception date received by the aforementioned receiving device;
a reception information storing device for associating and storing the patient information and inputted reception-related information, based on the information for separating the patients individually and information on reception date;
wherein the aforementioned medical image controlling apparatus acquires the image data and the information on the date of capturing the image data associated with patient information of the patient to be examined and stored in the image storing device; and obtains the examination information and/or treatment information together with the information on reception date, associated with the patient information and stored in the reception information storing device; the aforementioned medical image controlling apparatus containing a display device which associates the acquired image data with examination information and/or treatment information, and displays the acquired image data and these pieces of information in a list in chronological order on a screen, together with the patient information.

The invention described in Claim 10 is based on that described in Claim 9 further characterized in that the aforementioned reception-related information input device includes the aforementioned patient information input device.

The invention described in Claim 11 is based on that described in Claim 9 or 10 further including a plurality of types of the aforementioned image generating apparatuses, wherein:
the aforementioned image storing device associates the image data generated by the image generating apparatuses, the patient information on the patient whose image to be captured, the information on the date of capturing the image data, and information representing the type of the image generating apparatus with one another and stores them; and
the display device of the medical image controlling apparatus displays the image data in a list for each type of image generating apparatus.

The invention described in Claim 12 is based on that described in any one of the Claims 9 through 11 further characterized in that the screen wherein the image data, examination information and/or treatment information are displayed together with the patient information in a list in chronological order is configured in such a way as to be changed to a screen for displaying a detail of each captured image displayed on the screen.

### EFFECTS OF THE INVENTION

The invention of the present patent application provides a method for efficiently associating the image data generated by an image generating apparatus with patient information while a doctor is examining a patient.

According to the invention of the present patent application, when image data has been received from the image generating apparatus, notification of the reception is given. This arrangement permits a control mode to be selected in response to the reception of the image data.

According to the invention of the present patent application, the control mode can be selected every time image data has been received.

According to the invention of the present patent application, the medical imaging system provided with a plurality of image generating apparatuses efficiently associates the image data generated by an image generating apparatus with patient information while a doctor is examining a patient.

According to the invention of the present patent application, a control mode can be selected in conformity to the image generating apparatus sending the image data.

According to the invention of the present patent application, the received image data can be associated with the patient information on other patients than the patient to be diagnosed.

According to the invention of the present patent application, the examination data generated by an examination apparatus can be associated with the patient information with high efficiency while a doctor is examining a patient.

According to the invention of the present patent application, in medical practitioners, past examination history such as the captured image information, examination information and treatment information of the patient to be examined can be displayed in a list on the workstation by a simple input operation in the facility.

According to the invention of the present patent application, the input operations of a doctor in a doctor consultation room are reduced and the doctor is allowed to concentrate on the examination of a patient.

According to the invention of the present patent application, the past medical care history is displayed in an easy-to-understand manner. This arrangement permits the doctor to recollect the past medical care history quickly.

According to the invention of the present patent application, the doctor uses the medical image controlling apparatus to check a list of the past medical care history and detailed captured images. This eliminates the need to search for the film of the captured image of a patient, and the doctor is allowed to concentrate on the examination of a patient.

### BRIEF DESCRIPTION OF THE DRAWINGS

Fig. 1 is a diagram representing an example of the overall structure of the small-sized diagnostic system 1 of the present invention;
Fig. 2 is a diagram representing an example of the layout of each apparatus in medical facilities wherein the small-sized diagnostic system 1 of Fig. 1 is applied;
Fig. 3 is a major block diagram showing the functional structure of the control apparatus 3 applied to the small-sized diagnostic system 1 of Fig. 1;
Fig. 4 is a diagram showing an example of data storage of the reception DB 50 of Fig. 4;
Fig. 5 is a diagram showing an example of the medical care summary screen 351 given in a display section 35 of Fig. 3;
Fig. 6 is a diagram showing an example of the medical care summary screen 351 given in a display section 35 of Fig. 3;
Fig. 7 is a diagram showing an example of the captured image display screen 352 given in a display section 35 of Fig. 3;
Fig. 8 is a flow chart showing the process of associating the patient information to be carried out by the CPU 31 of Fig. 3;
Fig. 9 is a example of the patient information association screen 353 given in a display section 35 of Fig. 3;
Fig. 10 is a flow chart showing the process B of associating patient information to be carried out by the CPU 31 of Fig. 3;
Fig. 11 is a diagram showing an example of the association verification screen 355 given in the display section 35 of Fig. 3;
Fig. 12 is a diagram showing an example of the captured image list display screen 356 given in the display section 35 of Fig. 3;
Fig. 13 is a diagram showing the process of medical care summary display implemented in the control apparatus 3 of Fig. 1, and a medical care summary display sequence carried out in response thereto in the control apparatus 3, server 4 and reception computer 5;
Fig. 14 is a diagram showing an example of the image comparison screen 354 displayed when the image comparison screen button has been pressed in Fig. 8;
Fig. 15(a) is a diagram showing an example of the list registered at the reception desk in a conventional examination system; and
Fig. 15(b) is a diagram showing an example showing cassettes being registered in the list of Fig. 15(a) of a conventional examination system by a radiographing technician from the department of radiology.

### DESCRIPTION OF REFERENCE NUMERALS

1. Small-sized diagnostic system
2. Image generating apparatus
2a. Ultrasonic diagnostic apparatus
2b. Endoscope apparatus
2c. CR apparatus
21. Conversion apparatus
3. Control apparatus (medical image controlling apparatus)
4. Examination apparatus
5. Reception computer (reception apparatus)
50. Reception DB
51. Examination information DB
6. Network
7. Server
70. Image DB
31. CPU
32. RAM
33. Storage section
331. Temporary storing section
34. Input section
35. Display section
36. Communication section
37. Interface
38. Image DB
39. Examination information DB
40. Bus
351. Medical care summary screen
352. Captured image display screen
353. Patient information associating screen
354. Image comparison screen
355. Association verification screen
356. Captured image list display screen

### BEST MODE FOR CARRYING OUT THE INVENTION

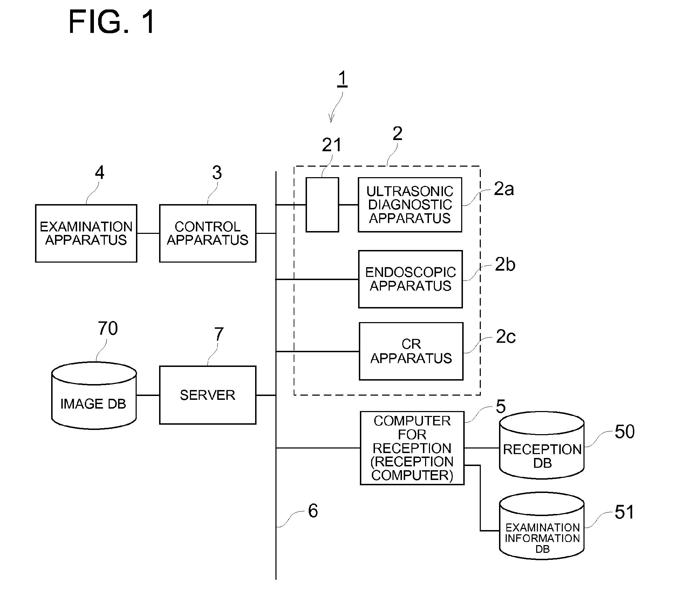

Referring to Figs. 1 through 11, the following describes an embodiment of the medical imaging system of the present invention, without the present invention being restricted thereto:
First, the structure will be described.

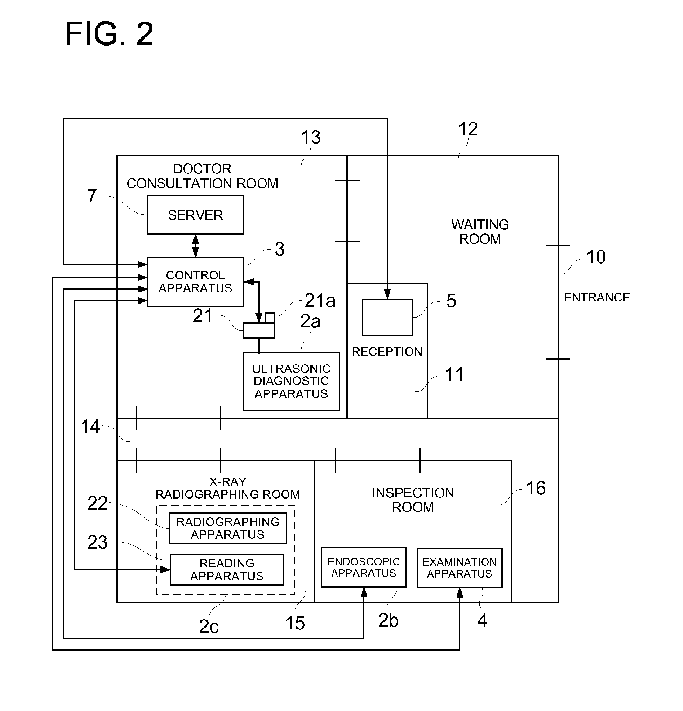

Fig. 1 shows the system structure of the small-sized diagnostic system 1 to which the medical imaging system of the present invention is applied. Fig. 2 shows the layout of each apparatus in medical facilities wherein the small-sized diagnostic system 1 of Fig. 1 is applied.

The small-sized diagnostic system 1 is a system applied to small-sized medical facilities of a medical practitioner or in a clinic. As shown in Fig. 1, the small-sized diagnostic system 1 includes an ultrasonic diagnostic apparatus 2a, endoscope apparatus 2b, CR apparatus 2c as the image generating apparatus 2, control apparatus 3 (also called the medical image controlling apparatus), examination apparatus 4, computer for reception 5 (hereinafter referred to as reception computer), and server 7. The image generating apparatus 2, control apparatus 3 and reception computer 5 are linked to the communication network 6 (hereinafter referred to as simply "network") such as LAN (Local Area Network) for example, through a switching hub (not illustrated). The control apparatus 3 is preferably a WS (workstation) installed in the doctor consultation room where a doctor is usually stationed. It is also possible to make such arrangements that the WS working as control apparatus 3 controls the startup of the image generating apparatus 2 and processing conditions.

The communication methods in a hospital are generally based on the DICOM (Digital Image and Communications in Medicine) Standards. The DICOM MWM (Modality Worklist Management) and DICOM MPPS (Modality Performed Procedure Step) are used in communication among various apparatuses linked by the LAN. It should be noted, however, that there are no particular restrictions to the communication methods that can be applied to the present embodiment.

For example, in small-sized medical facilities such as those of medical practitioners and in clinics, apparatuses are arranged as shown in Fig. 2.

When a visitor enters through the entrance 10, he will find a reception desk 11 for receiving a patient and a waiting room 12. The reception desk 11 is staffed by a person in charge of patient reception who assigns the outpatient with a reception number tag printed with the reception number for identifying patients in the order of reception. The reception desk 11 is provided with a reception computer 5. The person in charge of reception confirms the name of the patient and inputs the association between the reception number and patient name into the reception computer 5. After termination of the medical examination, the person in charge of reception also inputs the information on the request of insurance points (hereinafter referred to as "reception-related information"), based on the information in the patient medical chart.

Adjacent to the waiting room 12 there is a doctor consultation room 13 wherein the doctor examines and diagnoses the patient. For example, the examination desk (not illustrated) in the doctor consultation room 13 is provided with a control apparatus 3 and server 7 wherein the control apparatus 3 is used to display, for the doctor to diagnose, the captured image of the targeted examination region of the patient, examination information on the results of the examination of the patient (for example, sample test such as blood test, urine test, fecal test, tissue sampling test), and examination information on electrocardiography, and medical care information including the information on medication administered to the patient; and the server 7 is provided with the image DB (Database) 70 and is used to store the image data of the captured image. The doctor consultation room 13 is equipped with an ultrasonic diagnostic apparatus 2a characterized by a smaller need for use in an isolated space for the sake of privacy or the like.

An X-ray radiographing room 15 for X-ray radiographing is installed on the side opposite the doctor consultation room 13 across the corridor 14. A CR apparatus 2c made up of a radiographing apparatus 22 and reading apparatus 23 are installed inside the X-ray radiographing room 15. Further, an inspection room 16 is arranged adjacent to the X-ray radiographing room 15, and an endoscope apparatus 2b and examination apparatus 4 are installed in the inspection room 16.

The following describes the structure of each apparatus in the small-sized diagnostic system 1.

The image generating apparatus 2 is an image generating device (modality) including an ultrasonic diagnostic apparatus 2a, endoscope apparatus 2b, and CR apparatus 2c, wherein the image generating apparatus captures the image of the targeted examination region of the patient as a subject, converts the captured image into a digital image, and generates the image data of the captured image (captured image information).

The ultrasonic diagnostic apparatus 2a is made up of an ultrasonic probe (not illustrated) for outputting the ultrasonic wave, and an electronic apparatus (not illustrated), connected to the ultrasonic probe, for converting the sound wave (echo signal) received by the ultrasonic probe into image data of the captured images of the internal organs. The ultrasonic diagnostic apparatus 2a sends the ultrasonic wave from the ultrasonic probe into the patients body, and the sound wave (echo signal) reflected from the body organs is again received by the ultrasonic probe. The captured image in response to this echo signal is generated by the electronic apparatus.

The ultrasonic diagnostic apparatus 2a is connected with a conversion apparatus 21 as a converting device (converter) that converts the analog signal to a digital signal, and is connected with the network 6 through the conversion apparatus 21. Even when the data not conforming to the standards of other external devices (e.g. communication protocol) connected to the network 6 are outputted from the ultrasonic diagnostic apparatus 2a, use of the conversion apparatus 21 ensures data communication with the external device connected to the network 6 by adequate conversion.

In the present embodiment, the conversion apparatus 21 is equipped with a function for assigning the image data with the UID (unique ID) that specifies the image data in the small-sized diagnostic system 1 conforming to the DICOM Standard. The UID is made up of identification information (hereinafter referred to as "modality ID") of the image generating apparatus 2, and the numerals showing the information on time and date of image capturing. In the present embodiment, the modality ID includes information showing the type of the image generating apparatus 2.

The conversion apparatus 21 is provided with an input operation section 21a consisting of a numeric keypad, keyboard, and touch panel. The structure of the input operation section 21a is not restricted to the structure shown here. For example, it can be a card reader wherein the information on a card is read by insertion thereof. The input operation section 21a is used to input the search ID (reception number in the present embodiment) which is set associated with the patient as the subject of image capturing. The conversion apparatus 21 ensures that the search ID and the aforementioned UID inputted from the input operation section 21a are attached to the image data of the captured image.

The search ID can be defined as the identification information for identifying the patient as a target of image capturing when the captured image is searched for after the image capturing. In this case it corresponds to the reception number assigned to the patient at the time of reception. In the small-sized diagnostic system 1 of the present embodiment, image capturing of the patient is carried out without the examination order for the patient (image capturing order) being generated or issued in advance, digital image data is generated, and then the doctor conducts associations between the patient's information and captured image. The search ID is the ID used for this association.

For example, when capturing an image of a patient having the reception number "001" assigned at the reception desk 11, "001" is inputted as the search ID of the patient on the input operation section 21a in the case where the reception number is inputted as the search ID on the input operation section 21a.

In a medical practitioner's office and the like, the number of visiting patients per day is in the range of about 10 to 40. Two digits are sufficient for the serial number of the reception number showing the order of reception. There is no particular restriction to the input operation section 21a if this two-digit numeral can be inputted. Thus, a less costly numeric keypad is preferable.

The following describes an example of using the reception number as the search ID. As will be described below, the search ID is not restricted to the reception number.

If there is no need for continuous image capturing of a plurality of patients (when there is sufficient space separating patients), the doctor can easily associate the image data with the patient based on the UID made up of the modality ID plus information on date and time of image capturing attached to each image. The inputting of the search ID is not always required. In this case, each image generating apparatus 2 is set so as to start operation without the search ID being inputted.

The information which is attached to the captured image and is inputted from the input operation section 21a is not restricted to the search ID. It is also possible to make such arrangements that various forms of information capable of identifying the patient such as the name of the patient are inputted from the input operation section 21a. It is also possible to input the information identifying the type of image capturing operation such as a simple image capturing without a contrast medium or image capturing using a contrast medium. The search ID and other information inputted are attached to the image data of the captured image as the supplementary information such as header information. When the image data is sent to an external apparatus, these pieces of information are sent associated with the image data.

The endoscope apparatus 2b is formed by a small-sized image capturing device (not illustrated) attached to the front end of the flexible tube (not illustrated). The image capturing device is made up of an objective optical system including the optical lens, an image pickup section arranged at the image forming position of the objective optical system, and an illumination section that is made up of an LED (Light Emitting Diode) and others to provide required illumination for image pickup operation (these are not illustrated). The image pickup section is provided with a solid image pickup element such as a CCD (Charge Coupled Device) and CMOS (Complementary Metal-Oxide Semiconductor). When there is incoming light, it is subjected to photoelectric conversion and is changed into an electric signal corresponding to the amount of incoming light. In the objective optical system, light on the area illuminated by the illumination section is concentrated by an optical lens and an image is formed on the solid pickup element of the image pickup section. The light entering the solid pickup element is subjected to photoelectric conversion, and the image data of the captured image is output as the electric signal.

The radiographing apparatus 22 constituting the CR apparatus 2c has a radiation source (not illustrated) and applies radiation to the region of the patient to be examined, whereby a still picture is taken. At the time of radiographing, a radiographic image conversion medium incorporating a radiographic image conversion plate equipped with a stimulable phosphor sheet which stores radiation energy (where all are not illustrated), for example, is arranged in the area exposed to the radiation from the aforementioned radiation source. The amount of radiation in conformity to the radiation transmittance distribution at the targeted examination region with respect to the amount of radiation applied by the radiation source is accumulated on the stimulable phosphor layer of the stimulable phosphor sheet built into the radiographic image conversion medium. The radiographic image information at the targeted examination region is recorded on this stimulable phosphor layer.

When the radiographic image conversion medium having recorded the radiographic image information of the targeted examination region has been loaded, the reading apparatus 23 applies the excitation light to the stimulable phosphor sheet of the inserted radiographic image conversion medium. The stimulated light emitted from the sheet is subjected to photoelectric conversion, and the obtained image signal is input to an analog-to-digital conversion, whereby the image data of the captured image is generated. The CR apparatus 2c can be an apparatus made up of a radiographing apparatus 22 and reading apparatus 23 built as an integral structure.

The endoscope apparatus 2b and the reading apparatus 2 of CR apparatus 2c is provided with a function for assigning the aforementioned UID to the generated image data. The assigned UID is attached to the image data.

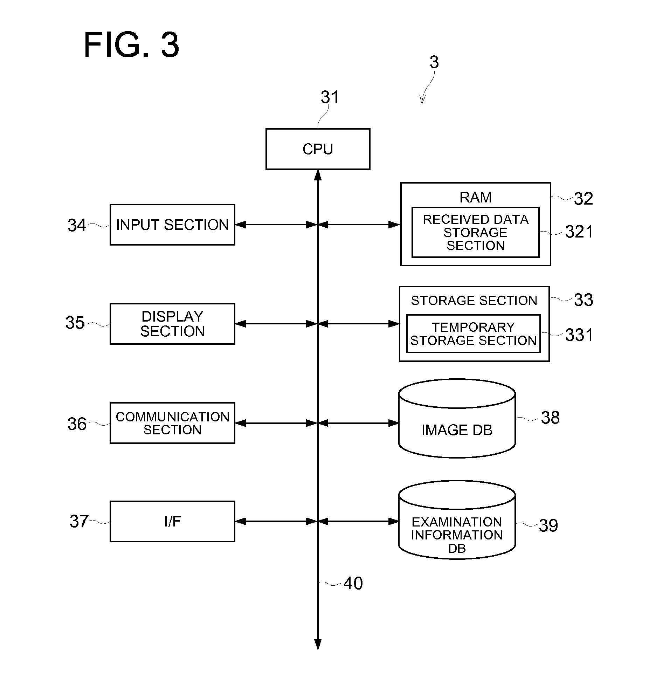

The control apparatus 3 is a medical image controlling apparatus which is installed, for example, in the doctor consultation room 13, and is used to associate patient information with image data and to store it in an image DB 38. The control apparatus 3 is also used by the doctor to display the image for captured image interpretation. The control apparatus 3 can be equipped with a monitor having a higher definition than that of the monitor (display) used in the general PC (personal computer). As shown in Fig. 3, the control apparatus 3 is provided with a CPU 31, RAM 32, storage section 33, input section 34, display section 35, and communication section 36, interface 37, image DB 38, and examination information DB 39. They are connected by buses 40.

The CPU 31 reads out various program forms including the system program and processing program stored in the control apparatus 33, loads them on the RAM 32, and implements various processing forms including the process of patient information association (to be described later) in conformity with the loaded program.

In various processing forms implemented and controlled by the CPU 31, the RAM 32 forms a work area for temporary storage of various program forms that are read out of the storage section 33 and can be run by the CPU 31, the input or output data, and parameters. In the present embodiment, the RAM 32 has a received data storage section 321 for temporary storage of the image data received from the image generating apparatus 2 and the examination data received from the examination apparatus 4.

The storage section 33 is formed of a HDD (Hard Disk Drive) and nonvolatile semi-conductor memory. The storage section 33 stores various forms of the aforementioned programs as well as the image processing parameters (such as a lookup table defining the gradation curve used for gradation processing, and the degree of enhancement in frequency processing) for adjusting the image quality suitable for diagnosis of the image data of the captured image.

The storage section 33 has a temporary storage section 331 as a temporary storage device which temporarily stores the image data and examination data which are not associated with the patient information.

The input section 34 includes a keyboard equipped with a cursor key, numeric input keys, and various function keys; and a pointing device such as a mouse. The depression signal of the keys having been depressed on the keyboard and the operation signal of input signals by the mouse are outputted to the CPU 31.

The display section 35 has monitors such as a CRT (Cathode Ray Tube) and LCD (Liquid Crystal Display). Various screens are displayed in conformity with the designation of the display signal inputted from the CPU 31.

The communication section 36 is formed of a network interface and others, and exchanges data with the external device connected to the network 6 through a switching hub.

The interface 37 is used to control data transmission and reception with the examination apparatus 4 by connecting the examination apparatus 4 to the control apparatus 3.

The image DB (Date Base) 38 contains an HDD and others, and provides a storage device for storing the image data of the captured image associated with the patient information.

The examination information DB 39 includes an HDD and others, and provides an examination information storage device for storing various forms of examination data associated with the patient information.

The examination apparatus 4 includes an electrocardiographic wave detecting apparatus for acquiring the waveform data by detecting the electrocardiographic wave; a vital sensor for acquiring the vital data such as body temperature, blood pressure, stature, and body weight; and a sound collector for acquiring the biological sound data such as pulmonary sound, cardiac sound, and voice. The acquired examination data (e.g., the aforementioned waveform data, vital data, biological sound data) is sent to the control apparatus 3.

### [Server and reception computer]

Returning to Fig. 1, the server 7 is a computer including a storage section made up of a CPU, RAM, and HDD; and a communication section for controlling the communications with various apparatuses connected to the network 6 (all not illustrated). The server is provided with the image DB 70. It implements association between the image data of the captured image whose writing is designated from the control apparatus 3 through the communications section, and its supplementary information (patient information and information including the UID), by software processing through collaboration with the programs stored in the CPU and storage section. Then the associated image data and the supplementary information are stored in the image DB 70 as an image storage device. At the same time, the server searches the image DB 70 in response to the request from the control apparatus 3, and reads out the image data and its supplementary information meeting the request. The image data and its supplementary information are then sent to the control apparatus 3.

The reception computer 5 is a computer equipped with a storage section made of the CPU, RAM, and HDD; an input section including a keyboard and mouse; a display section formed of a CRT and LCD; and a communication section (all not illustrated) for controlling communications with various apparatuses connected to the network 6. When the display on the reception input screen has been instructed from the input section, the reception computer 5 displays the reception input screen (not illustrated) on the display section by software processing through collaboration with the program stored in the CPU and storage section. When the reception information (reception number plus patient information) has been inputted by the input section through the reception input screen, the patient information list of the received patient is created (updated) and is stored in the storage section. This list is transmitted to the control apparatus 3 from the communication section in response to a request of the patient list transmission from the control apparatus 3.

Further, the reception computer 5 has a reception DB 50 for storing the reception-related information on the received patient that has been inputted by the operator in charge of the reception desk according to the paper patient medical chart information from the doctor; and an examination information DB 51 for storing the examination information on the examination conducted on the patient.

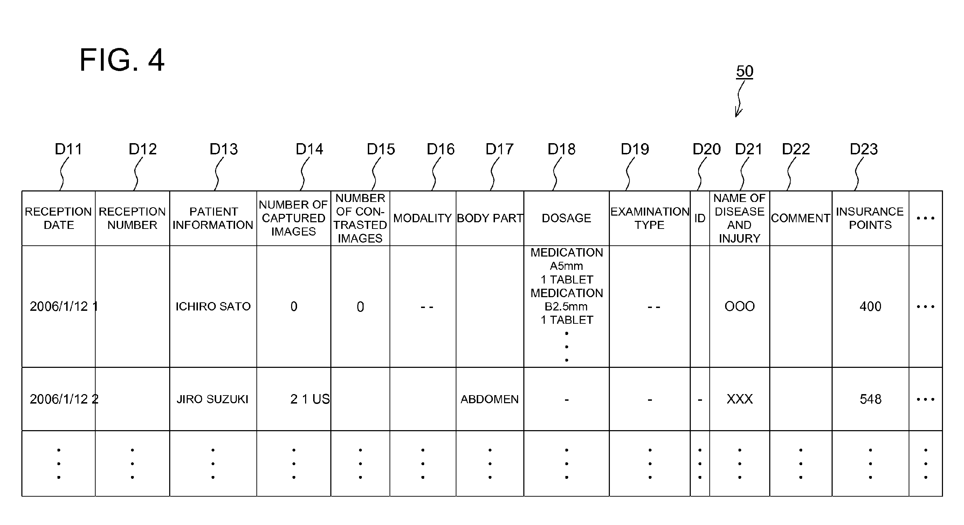

Fig. 4 shows an example of the data storage of the reception DB 50. The reception DB 50 is a database for storing the reception-related information of the visiting patient. As shown in Fig. 4, the reception DB 50 stores:
a "reception date" item D11 for storing the date on patient reception;
a "reception number" item D12 for storing the reception number assigned to the patient;
a "patient information" item D13 for storing the patient information (name of the patient in this case);
a "number of captured images" item D14 for storing the number of the images obtained from image capturing of the patient on the date of reception;
a "number of contrasted images" item D15 for storing the number of the images obtained from image capturing using a contrast medium;
a "modality" item D16 storing the type of image generating apparatus 2 used for image capturing;
a "body part" item D17 for storing the information on the image-captured region;
a "dosage" item D18 for storing the information on the medication prescribed for the patient on the date of reception;
an "examination type" item D19 for storing the information on the type of examination conducted on the patient;
an "ID" item D20 for storing the examination ID (sample ID in the case of sample test) to identify the examination conducted on the patient;
a "name of disease and injury" item D21 for storing the name of the disease or injury diagnosed by the doctor on the date of reception;
a "comment" item D22 for storing the comment inputted from the control apparatus 3 (to be described later); and
a "insurance points" item D23 for storing the calculated insurance points.

The examination information DB 51 is a database for storing the examination information showing examination results associated with the examination ID.

The reception DB 50 and examination information DB 51 in the present embodiment correspond to the device of reception information storage. In the present embodiment, to store detailed examination information and the examination information captured by a scanner, examination information is stored in the examination information DB 51 apart from the reception DB 50 and the reception DB 50 and examination information DB 51 are associated with each other through an examination ID, whereby they are both stored being associated. Since the examination information is the information contained in the reception-related information, the reception DB 50 can be provided with the "examination information" item, and the examination information indicating the result of examination can be stored in the "examination information" item.

When display on the reception input screen has been designated from the input section, the reception computer 5 displays the reception input screen (not illustrated) by the software processing through collaboration with the program stored in the CPU and storage section. When the reception information (reception number plus patient information) has been inputted from the input section through the reception input screen, a new record is added to the reception DB 50. The current date is written and registered in the "reception date" item, the inputted reception number in the "reception number" item, and the patient information in the "patient information" item. To put it another way, "reception device" and "patient information input device" are implemented by the reception input screen and the input section of the reception computer 5. When the display on the display section of the reception-related information input screen has been instructed from the input section, the reception-related information input screen (not illustrated) is displayed on the display section. When the reception number of the patient, as the object of inputting the reception-related information, has been inputted and the information on each item shown in Fig. 4 has been inputted on the reception-related information screen, the inputted information is written to the item corresponding to the record containing the current reception date and the reception number of the patient as the object of input, in the reception DB 50. To put it another way, the "reception-related information input device" is implemented by the reception-related information input screen and the input section of the reception computer 5. When the examination ID and examination information have been inputted from the input section through the examination information input screen (not illustrated), the inputted examination ID and examination information are associated with each other and are stored in the examination information DB 51. It is also possible to make such arrangements that the reception computer 5 is equipped with a scanner, and the examination information is inputted by reading the paper indicating the result of examination with the scanner. The reception computer 5 ensures that the information whose registration has been designated from the control apparatus 3 through the communication section is written to the corresponding item of the corresponding record of the reception DB 50 by software processing through collaboration with the program stored in the CPU and storage section. At the same time, the reception computer 5 searches the reception DB 50 in response to the request from the control apparatus 3, reads out the requested information, and sends (supplies) it to the control apparatus 3.

In the present embodiment, the reception number and patient information is inputted from the reception input screen at the time of reception. Only the reception number can be inputted at the time of reception, and the patient information can be inputted through the reception-related information input screen. To put it another way, it is also possible to make such arrangements that the reception-related information input screen includes the patient information input device. Of the patient information, only the name of the patient can be inputted from the reception-related information input screen. The other detailed patient information such as sex, age, and insurance number can be inputted from the reception-related information input screen.

The following describes the examination flow of a patient in the small-sized facilities wherein the small-sized diagnostic system 1 is applied:

When a patient enters the facility, he is assigned a reception number tag at the reception desk 11, and the reception number of the patient and the patient information including the patient name received by the operation at the input section are inputted (reception input) in the reception computer 5. When the patient reception number and patient information have been inputted in the reception computer 5, the patient information list is generated (updated) and is stored in a predetermined area of the RAM. The patient information list is generated, for example when the reception of the first patient of the day has been inputted, and is stored in the predetermined area of the RAM. The patient information list is updated every time a new patient is received.

When the patient assigned with a reception number goes into the doctor consultation room 13, the doctor begins a medical examination with an interview, and determines the image capturing and examination to be conducted.

When the need for image capturing of a part of the patient has been determined in the medical examination with an interview by the doctor, the image capturing technician or doctor who takes a picture takes the patient before the image generating apparatus 2 (ultrasonic diagnostic apparatus 2a, endoscope apparatus 2b, or CR apparatus 2c) for image capturing, and takes a picture of the targeted examination region of the patient, whereby the image data of the captured image is generated. For example, when the CR apparatus 2c is used as the image generating apparatus 2, a radiograph is taken by the radiographing apparatus 22 and the cassette having been irradiated by the radiographing apparatus 22 is set on the reading apparatus 23. The radiation image recorded on the cassette is read out. In the image generating apparatus 2, radiographing and generation of the image data is performed in response to the operation of the person who takes a radiograph. The aforementioned UID is attached to the image data having been generated, and the image data is transmitted to the control apparatus 3.

When the need for measurement of the blood pressure and body temperature, and electrocardiographic examination has been determined by a medical examination interview with a doctor, the doctor or nurse examines the patient using the examination apparatus 4. The examination data obtained by the examination apparatus 4 is transmitted to the control apparatus 3.

Upon completion of radiographing and examination, the doctor uses the control apparatus 3 to display the captured image received from the image generating apparatus 2 and the examination results sent from the examination apparatus 4, whereby the doctor examines the patient referring to the captured images or examination results.

The above description provides a major flow for the examination of a patient. The image data generated by the image generating apparatus 2 and the examination data obtained by the examination apparatus 4 are associated with the patient information by the control apparatus 3. To assist the doctor, the control apparatus 3 displays the captured image and examination results based on the received image data and examination data. Accordingly, during the examination of the patient, the captured image and examination results of the examined patient obtained on this date or previous dates are kept displayed.

The captured image and examination results of the examined patient obtained on this date or previous dates are displayed, for example, on the medical care summary screen 351 or captured image display screen 352 (to be described later).

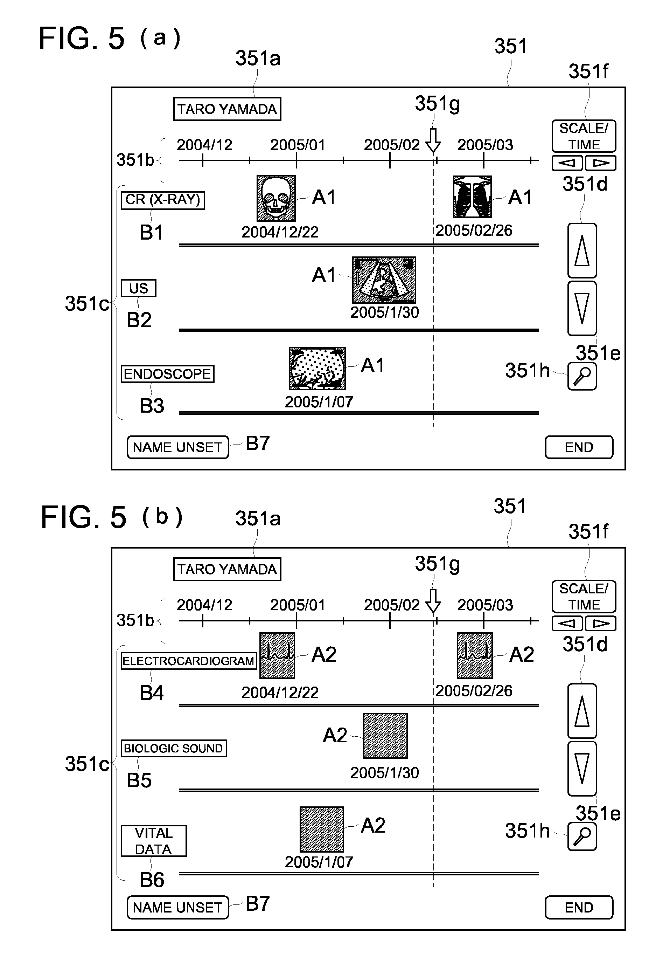

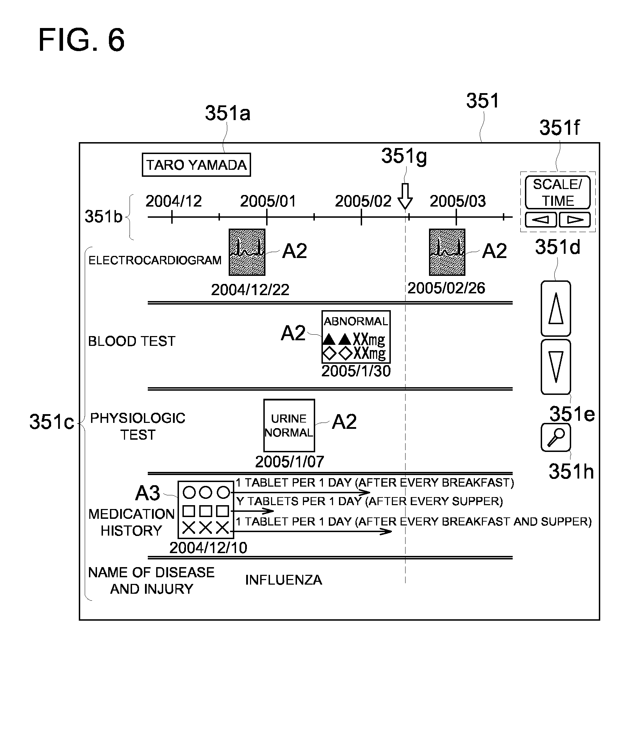

Figs. 5 and 6 show an example of the medical care summary screen 351. The medical care summary screen 351 is the screen wherein the captured images and examinations conducted on the patient designated by the input section 34 are displayed in a list in chronological order. The medical care summary screen 351 includes patient information 351a, time axis 351b, medical care summary display area 351c, upward scroll button 351d, downward scroll button 351e, arrow icon 351g, tack-pin icon 351h, and scale/time change area 351f for designation of change of time axis display scale and display time. The object icon A1 being a thumbnail representation of the image captured from the designated patient is displayed while being classified for each image generating apparatus 2 and being associated with the time axis 351b in the medical care summary display area 351c. The object icon A2 notifying the completion of acquiring the examination results (hereinafter referred to as "examination icon") is classified for each examination apparatus 4 and is displayed at a position in the time axis 351b of the medical care summary screen 351 corresponding to the date when the examination data is obtained.

The patient information of the patient to be displayed as the object on the medical care summary screen 351 is displayed in the patient information area 351a. A time axis for the time period is preset as a default, for example, the time period up to now from "N" months ago is displayed in the time axis area 351b. The medical care summary display area 351c shows the object icon A1 showing the image data acquired at the same type of modality within the time period of the displayed time axis; the object icon A2 indicating the examination information of the same examination item examined within the time period of the displayed time axis; a object icon A3 showing the medication dosage information within the time period of the displayed time axis; and the name of the disease and injury determined by the doctor, wherein they are displayed across the screen corresponding to respective time axes. These object icons A1 through A3 provide the doctor with easy access to the image captured by photographing the patient, the examination information on the examination conducted on the patient, and the medical cure information on the medical treatment that applies to the patient.

When there is a great deal of information to be displayed on the medical care summary display area 351c and one screen is not sufficient to display it, scroll buttons (upward scroll button 351d and downward scroll button 351e) appear. The information on the medical care summary display area 351c is displayed by scrolling in response to the operation of pressing each scroll button from the input section 34. This procedure allows the information not currently displayed to appear. For example, in the medical care summary screen 351 of Fig. 5(a), when the downward scroll button 351e is pressed from the input section 34, the information of the medical care summary display area 351c is scrolled down, whereby the examination information and treatment information are displayed, as shown in Fig. 5(b) or Fig. 6.

The scale/time change area 351f for designation of change of the time axis display scale and display time in the medical care summary screen 351 has a scale/time selection area, left arrow button, and right arrow button. Through collaboration with the input section 34, they serve as a display time setting device for setting the time period of the time axis displayed on the display screen. In response to the selection operation of the scale or display time (time) in the scale/time selection area from the input section 34, the function of the right arrow button and left arrow button is switched over to the change of the display scale or change of the display time. For example, when the "Scale" has been selected from the input section 34, the right arrow button and left arrow button are switched over to the change instruction input button of the display scale of the time axis. The display scale of the time axis is reduced in response to the subsequent operation of pressing the left arrow button from the input section 34, and the display scale of the time axis is enlarged in response to the operation of pressing the right arrow button. When the "Time" has been selected from the input section 34, the right and left arrow buttons are switched over to the change instruction input button of the display time of the time axis. In response to the subsequent operation of pressing the left arrow button from the input section 34, the display time of the time axis is changed over to the past side. In response to the subsequent operation of pressing the right arrow button from the input section 34, the display time of the time axis is changed over to the present side. This is followed by extraction of the image data, examination information, and medication dosage information within the range of the displayed time axis. Based on the extracted information, object icons A1 through A3 are created and are displayed at the position corresponding to the time axis. Further, when a point on the time axis has been designated by the mouse, the period of one month including the point designated on the time axis is displayed as the time axis, and the image data, examination information, and medication dosage information for the designated time period of one month are displayed being correlated with the time axis. For the medical treatment information shown by the object icon A3, the medical treatment (medication) time period is preferably displayed being correlated with the time axis, as shown in Fig. 6. Further, the size of each object icon is adjustable to ensure easy viewing of a display list in conformity with the time axis scale.

Further, on the medical care summary screen 351, the arrow icon 351g for setting the reference time on the time axis is displayed. The time on the time axis indicated by the arrow of the arrow icon 351g is set to the reference time. When the arrow icon 351g is dragged horizontally across the screen by the mouse of the input section 34, the reference time on the time axis conforms to the dragging operation. To put it another way, a reference time setting device for setting the reference time on the time axis is implemented by the operation of the input section 34 to the arrow icon 351g. As shown in Figs. 5(a) and 5(b) and Fig. 6, the reference time designated by the arrow icon 351g is indicated by a dotted line over the entire screen. This provides the doctor with easy access to a change in the state of a disease of the patient before and after this reference time. For example, if the time for changing the prescription of the medication is set to the reference time, the doctor can easily understand the effects of medical treatment before and after a change in the prescription of the medication by comparison of the image and examination information before and after the reference time. If the arrow icon is clicked by the mouse, the comment column is displayed and comments on the reference time can be inputted. When a comment has been inputted from the comment column (to be described later) by the input section 34, the comment information inputted into the comment item of the reception-related information of the reception DB 50 corresponding to the date designated by the arrow icon is written. When the arrow icon is set at the position thereafter corresponding to this date, the command appears close to the arrow icon 351g.

When any one of the object icons A1 displayed on the medical care summary display area 351c have been designated by the input section 34 representing an icon designation device, the display of the display section 35 is switched to the captured image display screen 352 that shows the details of the image shown by the object icon by the control.

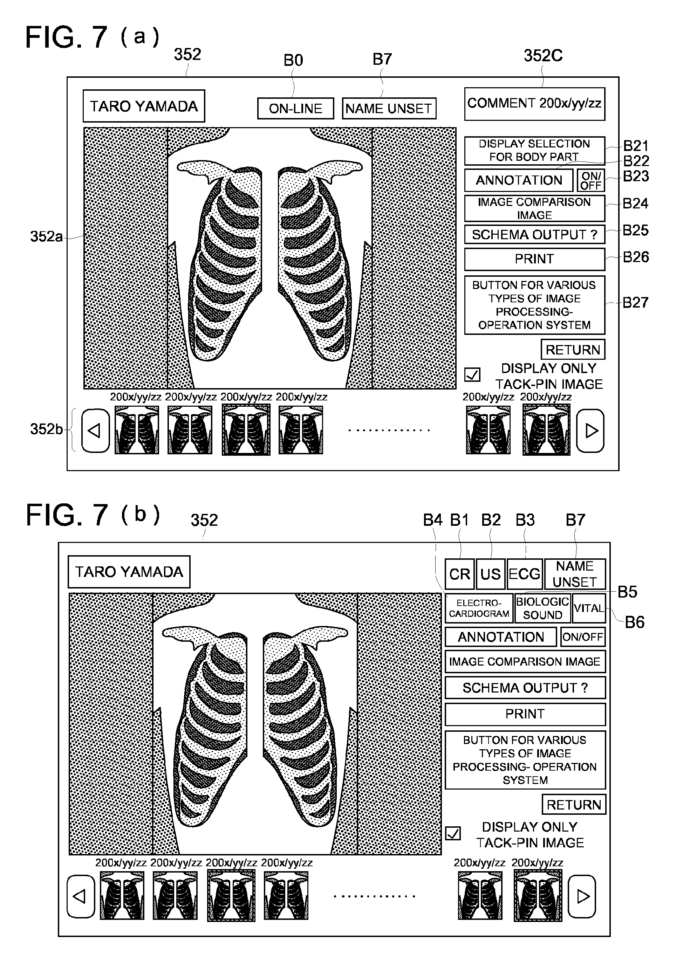

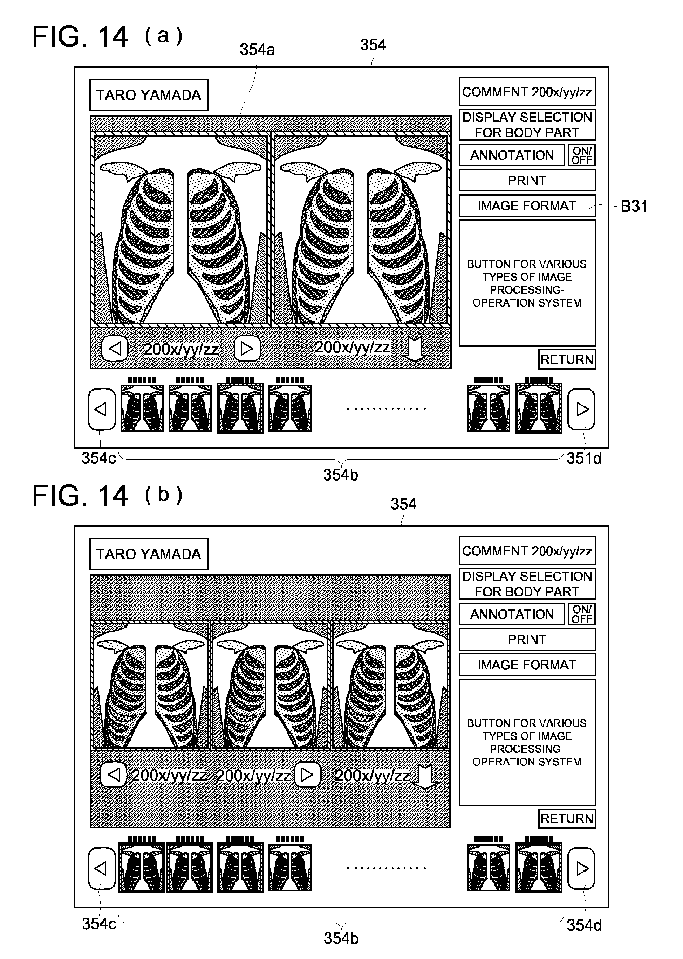

Fig. 7(a) shows an example of the captured image display screen 352. The captured image display screen 352 is a screen used by the doctor to diagnose the image. In the present embodiment, it is a screen displayed by operating the medical care summary screen 351 giving a list display thereof, by thumbnail representation of the image data and examination information. The captured image display screen 352 has an image display area 352a for displaying the image designated on the medical care summary screen 351; a photographing sequence display area 352b wherein thumbnail representations of the images of the aforementioned patient at the same modality are displayed in the order of photographing; and a comment display column 352c for displaying the comment inputted with respect to the reference time designated by various forms of operation buttons and on the medical care summary screen 351. In the captured image display screen 352, when the "display selection for body part" button B21 has been pressed from the input section 34, the photographed regions of the aforementioned patient are displayed in a list. When the region is selected from the list display, only the captured image of the selected region is displayed on the captured image display screen 352. When the "annotation" button B22 has been pressed from the input section 34, various forms of annotations including the arrow mark and solid mark can be inputted. When the ON/OFF button B23 has been pressed from the input section 34, the annotation display/non-display is switched. When the "image comparison screen" button B24 has been pressed from the input section 34, the display of the display section 35 is switched over to the image comparison screen 354 (Fig. 14, to be described later). If the "schema output" button B25 has been pressed, the control goes to the process of pasting the image and annotation displayed on the image display area 352a to the designated electronic patient medical chart, when the control apparatus 3 is linked with the electronic patient medical chart DB for storing the electronic patient medical chart. When the "print output" button B26 has been pressed, the image displayed on the image display area 352a is outputted. Processing in response to the operation of pressing is applied to the image data displayed on the image display area 353a by pressing the operation button B27 for various forms of image processing (enlargement/reduction, rotation/reversing, gradation adjustment, and density adjustment).

At the time of a medical examination interview, the doctor uses the control apparatus 3 to designate the patient to be examined, displays the medical care summary screen 351 and captured image display screen 352 of that patient, and determines the number of captured images and direction of photographing. Then, the doctor requests an image capturing technician to perform image capturing or performs an examination. Thus, in principle, there is agreement between the patient designated by the control apparatus 3 and the patient of the received image data and examination data. However, when there is an emergency patient, there is a follow-up examination wherein image capturing (examination) can be performed under the same image capturing (examination) conditions, or when the hospital is crowded with patients, there is no agreement between the patient to be examined currently designated by the control apparatus 3 and the patient of the received image data or examination data.

Thus, the control apparatus 3 has two modes such as an on-line mode (first control mode) wherein the received image data and examination data is automatically associated with the designated patient information, and is stored, and an off-line mode (second control mode) wherein, without the received image data and examination data being associated with the designated patient information and they are temporarily stored in the temporary storage section 331 in such a way that they can be associated with the designated patient information later. The selection (switching) between two modes can be controlled on the aforementioned medical care summary screen 351 and captured image display screen 352. Control is provided in conformity with the inputted selection operation by patient information association processing, which will be described below.

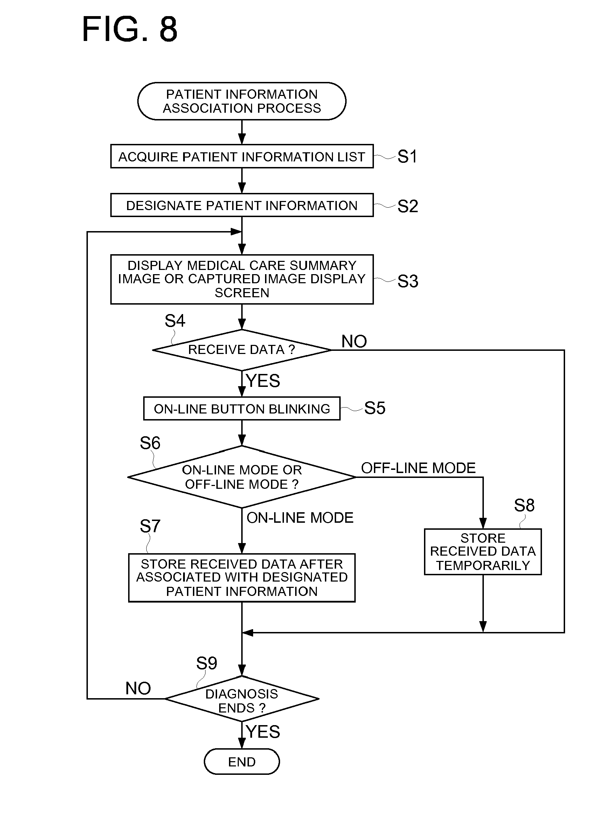

Fig. 8 shows the patient information association processing. This processing is implemented by software processing through collaboration of the CPU 31 with the patient information association processing program stored in storage section 33, when there is an input of a predetermined operation to instruct the display of the patient designation screen (not illustrated) for designating of the patient to be examined.

The patient list transmission request is sent to the reception computer 5 through the communication section 36, and the patient information list data is acquired from the reception computer 5 (Step S1: patient list acquisition device). This is followed by the step of displaying the patient designation screen showing the acquired patient information list on the display section 35. When the patient information of the patient representing the target of examination is specified from the input section 34 through this screen (Step S2: patient designation device), the image data stored being associated with the information on the patient designated by the image DB 38 of the control apparatus 3 is extracted. At the same time, the examination data stored being associated with the information on the patient designated by the examination information DB 39 is extracted. The display section 35 shows the medical care summary screen 351 which displays the thumbnail screen A1 of the extracted image data and examination icon A2 of the extracted examination data. When the display instruction of the captured image display screen 352 has been inputted from the input section 34 through the medical care summary screen 351, the captured image display screen 352 is displayed on the display section 35 (Step S3).

After the medical care summary screen 351 or captured image display screen 352 have been displayed, when the image data from the image generating apparatus 2 has been received by the communication section 36 or the examination data from the examination apparatus 4 has been received by the interface 37, and the received data has been stored in the received data storage section 321 (YES in Step S4), the on-line button representing a notifying device for notifying the reception of signals blinks (Step S5). In the medical care summary screen 351, as shown in Fig. 5, the on-line buttons B1 through B3 for each image generating apparatus 2 (US corresponds to the ultrasonic diagnostic apparatus 2a) and the on-line buttons B4 through B6 for each examination apparatus 4 are displayed. In Step S4, the on-line button corresponding to the image generating apparatus 2 or examination apparatus 4 as the data transmitter blinks. In the captured image display screen 352, as shown in Fig. 7(a), one on-line button is shown. When the data has been received by the communication section 36 or interface 37, the on-line button B0 blinks. It is also possible to make such arrangements that, in the captured image display screen 352, as shown in Fig. 7(b), the on-line buttons B1 through B3 (ECG corresponds to endoscope apparatus 2b) for each image generating apparatus 2, and the on-line buttons B4 through B6 for each examination apparatus 4 are displayed. The on-line button corresponding to the data transmitter may be allowed to blink. If data is not received in Step S4 (NO in Step S4), processing goes to Step S9.

The on-line button functions as a control mode selection device. If it is pressed when it blinks, the operation instruction in the on-line mode is outputted to the CPU 31. If it is kept depressed for an extended period of time (e.g., for 10 seconds or more), the operation instruction in the off-line mode is outputted to the CPU 31. If the image data has been received from the image generating apparatus 2, the on-line button blinks. This makes it possible for the doctor to select (switch) the control mode in response to reception of the image data or examination data.

When the blinking on-line button is pressed by the mouse of the input section 34 and the operation instruction in the on-line mode has been inputted (Step S6: on-line mode), and if the received data is the image data, this image data is associated with the patient information designated in Step S2, and is stored in the image DB 38. If the received data is the examination data, this examination data is associated with the patient information designated in Step S2, and is stored in the examination information DB 39 (Step S7). Then, the processing goes to Step S9. When the medical care summary screen 351 is displayed on the display section 35, the object icon A1 for thumbnail representation of the received image data or the examination icon A2 of the received examination data are generated. They are displayed at the position of the medical care summary screen corresponding to the time axis. In the meantime, when the blinking on-line button is kept depressed for an extended period of time (e.g., for 10 seconds or more) by the mouse of the input section 34, and the operation instruction in the off-line mode is inputted (off-line mode in Step S6), the received data (image data or examination data) is associated with the number showing the order of reception and is temporarily stored in the temporary storage section 331 (Step S8). Processing then goes to Step S9. When the data is temporarily stored in the temporary storage section 331, the data is preferably classified for each image generating apparatus 2 and each examination apparatus 4 representing the transmitter.

If there is no input of instruction in Step S9 (No in Step S9) to terminate the examination of the patient designated from the input section 34 in Step S2, processing goes back to the Step S4. If there is an input of instruction to terminate the examination of the designated patient from the input section 34 (Yes in Step S9), this processing terminates.

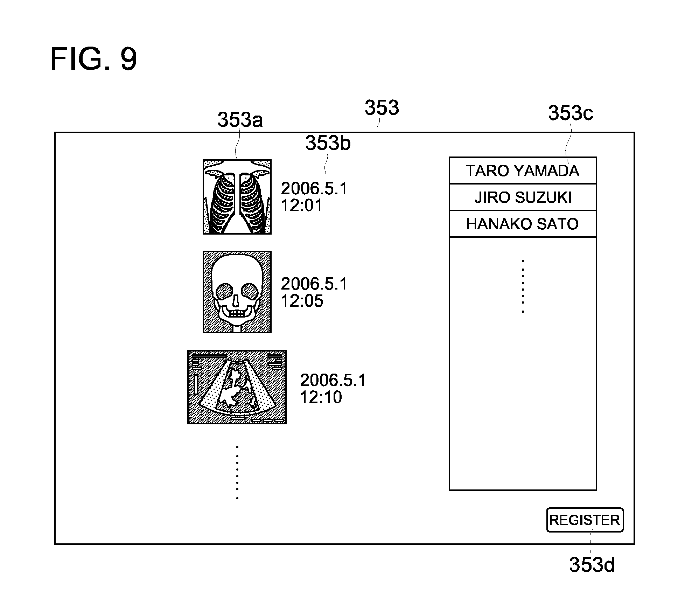

The image data and examination data stored in the temporary storage section 331 can be associated with the patient information on the patient information association screen 353 displayed when the name unset button B7 displayed on the medical care summary screen 351 or captured image display screen 352 is pressed. Fig. 9 shows an example of the patient information association screen 353. As shown in Fig. 9, the thumbnail image 353a and the examination icon for examination data generated based on the image data and examination data stored in the temporary storage section 331 are associated with its acquisition time 353b (e.g., UID attached to each image data) and are displayed in the chronological order of the acquisition on the patient information association screen 353. The patient information list 353c is also displayed on the patient information association screen 353. It is also possible to adopt such a structure wherein these pieces of data are displayed in the order of being received by the control apparatus 3, not in the chronological order in which they are acquired. The thumbnail image and examination icon are selected from this screen, and the patient information corresponding to the selected thumbnail image and icon is selected from he patient information list. When the "register" button 353d has been pressed, the image data corresponding to the selected thumbnail image is associated with the selected patient information and is stored in the image DB 38. The examination data corresponding to the selected icon is associated with the selected patient information and stored in the examination information DB 39.

As described above, if the image data or examination data has been received in the control apparatus 3 while the screen related to the designated patient information is displayed, the on-line button blinks. When the blinking on-line button is pressed, the received data is automatically associated with the designated patient information and is stored in the image DB 38. When the blinking on-line button is kept depressed for on extended period of time, the received data is stored in the temporary storage section 331 for storing the data waiting to be associated with the patient information.

Thus, the doctor designates the information on the patient to be examined on the control apparatus 3 and makes diagnosis by displaying the captured image and the examination results having been obtained in the past from the patient or the captured image and the examination results conducted on this patient just before. During this time, the doctor receives the captured image from the image capturing technician who has been requested to provide such an image and data on the results of examination for this patient. In this case, the doctor can press the on-line button so that the received image data is automatically associated with the patient information on the designated patient and is stored in the image DB 38. If the doctor receives more pieces of image data or more pieces of examination data on this patient than those which is supposed to be supplied by the image capturing technician according to the previous request, the doctor keeps the on-line button depressed for an extended period of time so that the received data is stored in the temporary storage section which stores the data waiting to be associated with the patient information. This data can be associated with the patient later. This arrangement ensures association of the patient information and the storage operation by simple switching in response to the particular circumstances of the patient in the hospital. Thus, while diagnosing the patient, the doctor is able to perform efficient associations between the patient information and the image data and examination data of the medical image generated by the image generating apparatus 2. Further, as shown in Fig. 7(b), if the on-line button is displayed for each image generating apparatus 2 or examination apparatus 4 being linked, the doctor can easily identify from which of the image generating apparatuses 2 the data has been sent. This arrangement ensures switching of association of the image data with the patient information and the storage operation to be performed in response to the transmitter of the transmitted data. This arrangement enhances maneuverability.

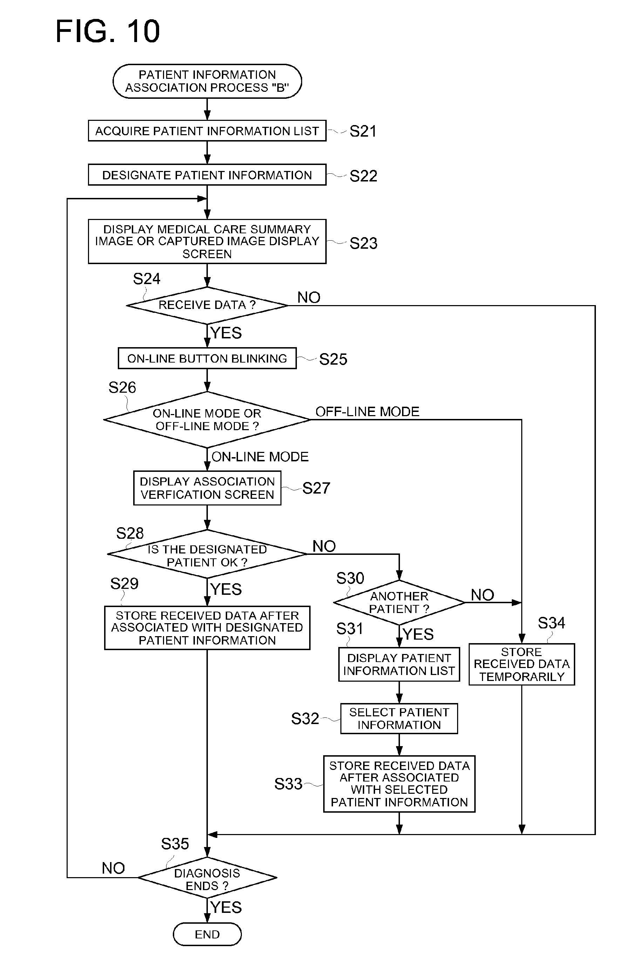

It is also possible to adopt such a structure that, if the data of another patient has been received during the display of the medical care summary screen 351 and captured image display screen 352 corresponding to the designated patient information, association with another patient can be achieved on the spot. Referring to Fig. 10, the following describes the variation of the aforementioned embodiment. This example of a variation permits the received data to be associated with the patient information of a patient other than that of the patient currently being examined.

Fig. 10 shows the flow chart of the patient information association processing B as an example of the variation of the patient information association processing described with reference to Fig. 8. This processing is implemented by software processing collaboration with the CPU 31 and the program stored in storage section 33, when there is an input of a predetermined operation for designating the display of the patient designation screen for designating the patient to be examined.

In the first place, a patient list transmission request is sent to the reception computer 5 through the communication section 36, and the patient information list data is acquired from the reception computer 5 (Step S21). This is followed by the step displaying the patient designation screen showing the patient information list acquired on the display section 35. When the patient information of the patient to be examined is specified from this screen through the input section 34 (Step S22), the image data stored being associated with the patient information designated in the image DB 38 is extracted. At the same time, the examination data stored being associated with the patient information designated in the examination information DB 39 is extracted. This is followed by the step displaying the medical care summary screen 351 showing the thumbnail image A1 of the extracted image data and the examination icon A2 of the extracted examination data onto the display section 35. Further, if the instruction to display the captured image display screen 352 is inputted from the medical care summary screen 351 through the input section 34, the captured image display screen 352 appears on the display section 35 (Step S23).

After the display of the medical care summary screen 351 or captured image display screen 352, when the image data from the image generating apparatus 2 is received by the communications section 36, or the examination data from the examination apparatus 4 is received by the interface 37, the received data is stored in the received data storage section 321 (Yes in Step S24), and the on-line button blinks (Step S25). If the data has not been received in Step S24 (No in Step S24), the processing then goes to Step S35.

In Step S26, when the blinking on-line button is kept depressed for an extended period of time by the mouse of the input section 34 and the operation instruction in the off-line mode has been inputted (off-line mode in Step S26), the processing goes to Step S34 and the received data (image data or examination data) is associated with the number showing the sequential order number of data reception and is temporarily stored in the temporary storage section 331. Then the processing goes to Step S35.

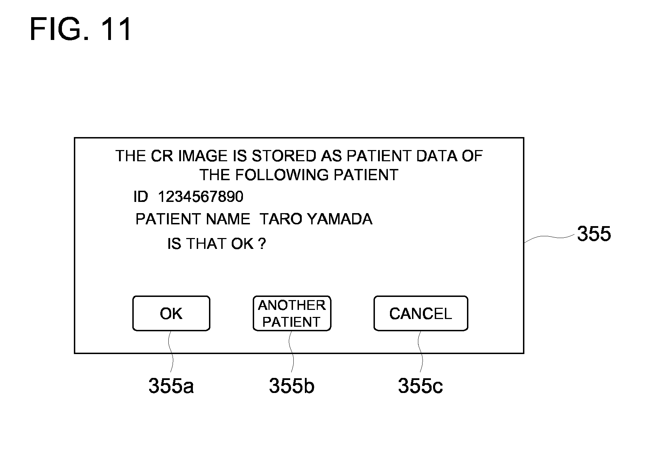

The blinking on-line button is pressed by the mouse of the input section 34, and the operation instruction of the on-line mode has been inputted (on-line mode in Step S26), the association verification screen 355 of Fig. 11 pops up on the display section 35 (Step S27). The association verification screen 355 shows the patient information of the currently designated patient, a message asking for verifying the suitability of association of the data with the displayed patient, OK button 355a, "other-patient" button 355b, and "cancel" button 355c. Similarly to the on-line button, these buttons function as a control mode selection device. The "other-patient" button 355b is used to select the mode (third control mode) for association of the received image data or examination data with patient information other than the designated patient information.

If the OK button 355a has been pressed on the association verification screen 355 to input the instruction to associate the received data with the designated patient information (Yes in Step S28), when the received data is an image data, the image data is associated with the patient information designated in Step S22 and is stored in the image DB 38. When the received data is an examination data, the examination data is associated with the patient information designated in Step S22 and is stored in the image DB 39 (Step S29). Then processing goes to Step S35. When the medical care summary screen 351 is displayed on the display section 35, the thumbnail image A1 of the received image data or the examination icon A2 indicating the examination data is generated, and is displayed at the position corresponding to the time axis of the medical care summary screen 351.

When the "other-patient" button 355b is pressed on this screen without the OK button 355a being pressed (No in Step S28) and the instruction for association with another patient has been inputted (Yes in Step S30), the patient information list is displayed on the display section 35 (Step S31). When the patient information as an object for association has been selected (Step S32), if the received data is an image data, the image data is associated with the patient information selected in Step S32 and is stored in the image DB 38. If the received data is an examination data, the examination data is associated with the patient information selected in Step S32 and is stored in the image DB 39 (Step S33). Then the processing goes to Step S35.

When the "cancel" button 355c is pressed on this screen without the "other-patient" button 355b being pressed, and the instruction to cancel the on-line mode has been inputted (No in Step S30), the processing goes to Step S34, and the received data (image data or examination data) is associated with the number showing the sequential order number of data reception, and is stored temporarily in the temporary storage section 331. Then the processing goes to Step S35.

In Step S35, the processing goes to Step S23 if the instruction to terminate the examination of this patient designated from the input section 34 in Step S22 is not inputted. If there is an instruction from the input section 34 to terminate the examination of the designated patient (Yes in Step S35), the processing terminates.

According to patient information association processing B, when the operation of the on-line button has been designated, not only the currently designated patient but also another patient can be associated. This arrangement enhances convenience.

The description in the aforementioned embodiment provides a preferred example of the small-sized diagnostic system 1 of the present invention, without the present invention being restricted thereto.

For example, in the aforementioned embodiment, the on-line mode or off-line mode can be selected by pressing the blinking on-line button or keeping it depressed for an extended period of time. Without the present invention being restricted thereto, for example, it is also possible to make such arrangements that an off-line button is separately installed, and the off-line button is pressed when the off-line mode is to be selected.

In the aforementioned embodiment, the image generating apparatus 2 has been described as an ultrasonic diagnostic apparatus 2a, endoscope apparatus 2b, and CR apparatus 2c. Without the present invention being restricted thereto, the image generating apparatus 2 can be a CT apparatus or a digital camera to photograph the external appearance of the body such as skin.

### [Another embodiment]

Mainly referring to Figs. 1 through 3 and Figs. 12 through 14, the following describes the procedures for generating captured images and reception-related information in the small-sized diagnostic system 1 of another embodiment.

At the reception desk 11, a visiting patient is given a reception number tag, and the name of the patient is recorded. Then, the patient information including the reception number and patient's name are inputted into the reception computer 5 from the input section through a reception input screen (not illustrated). A new record is added to the reception DB 50, and the reception date, reception number and patient information are written.

When the patient assigned with the reception number enters the doctor consultation room 13, the doctor begins a medical examination interview and determines image capturing and examination to be conducted.

When the need of capturing an image of a part of the patient has been determined from the medical examination interview by the doctor, the image capturing technician or doctor who takes a picture takes the patient before the image generating apparatus 2 (ultrasonic diagnostic apparatus 2a, endoscope apparatus 2b, or CR apparatus 2c) for image capturing, and inputs the search ID indicated on the reception number tag of the patient into the image generating apparatus 2 through the input operation section. The image capturing technician or doctor then takes a picture of the targeted examination region of the patient as an object, whereby the image data of the captured image is generated. For example, when the CR apparatus 2c is used as the image generating apparatus 2, the search ID is inputted from the numeric keypad of the input section of the reading apparatus 23. The cassette having been irradiated by the radiographing apparatus 22 is set on the reading apparatus 23. The radiation image recorded on the cassette is read out.

In the image generating apparatus 2, the numeric keypad of the input operation section is pressed and the search ID is inputted. Then image capturing is performed and image data is generated in response to the operation of the image capturing technician, and the inputted search ID and UID are attached to the generated image data, which is then sent to the control apparatus 3. When a plurality of images of one patient are captured under a plurality of conditions, the search ID and UID showing the reception number are attached to all pieces of the captured image data, which are then sent to the control apparatus 3. In the image generating apparatus 2, image data can be generated only when there is an input of the search ID. In case of emergencies, when capturing an image of a patient not yet accepted through the reception, a predetermined code (e.g., "99") indicating the emergency interruption that does not show up in the regular examination is set in advance. This procedure permits the image data to be extracted after image capturing, similar to the case of regular flow.

In the control apparatus 3, when image data (including the supplementary information) has been received from the image generating apparatus 2, the received image data is temporarily stored in the temporary storage section 331. Not only the image data of the relevant patient, but also pieces of image data obtained by capturing images of other patients without being interpreted are associated with the search ID and are accumulated in the temporary storage section 331 one after another.

Upon completion of image capturing and examination, the doctor examines the patient. When image capturing has been performed by the image generating apparatus 2, the image and patient are associated with each other and image interpretation is conducted, as described below.

The doctor operates the input section 34 of the control apparatus 3 to display the image search screen (not illustrated) on the display section 35. Checking the search ID on the reception number tag of the patient, the doctor inputs the search ID through the input section 34. In the control apparatus 3, the image search screen is displayed in response to the operation of the input section 34 and the input of the search ID is received through the input section 34. The image search screen is displayed. When the search ID is inputted from this screen through the input section 34, the image data corresponding to the inputted search ID is extracted from the temporary storage section 331, and image processing such as gradation processing or frequency enhancement processing is applied to the extracted image data. This procedure generates a thumbnail image which is obtained by reducing the size of the processed image data. This image is displayed on the captured image list display screen 356 of the display section 35.

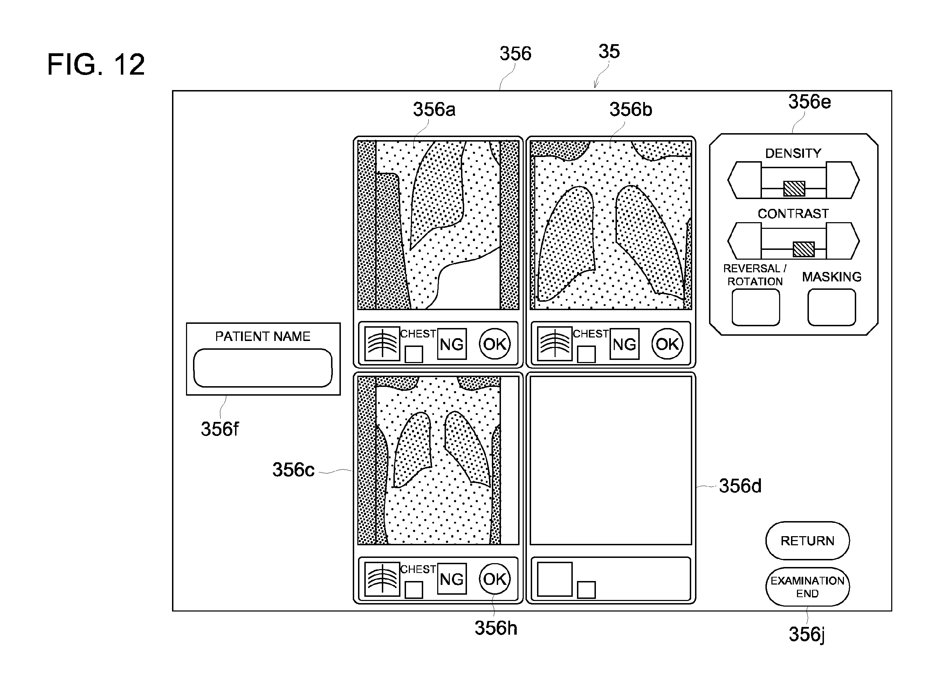

Fig. 12 shows an example of the captured image list display screen 356 shown in the display section 35. As shown in Fig. 12, the captured image list display screen 356 contains the image display columns 356a through 356d for list displays of the extracted images. When any one of the image display columns 356a through 356d has been selected by the mouse, the selected image is independently displayed in life-sized form. This independently displayed image permits the doctor to observe the details of the image and to perform image interpretation. The image processing adjustment column 356e is provided on the upper right corner of the screen. The doctor uses the mouse to operate this image processing adjustment column 356e so that the density and contrast are adjusted. When the OK button 356h displayed corresponding to each image, is pressed, the displayed image can be determined as the image to be stored in the image DB 70. The captured image list display screen 356 contains a patient information input column 356f for receiving the input of patient information such as the name of the patient to be associated with the displayed captured image. The doctor inputs the patient information from this patient information input column 356f.

When the patient information on the relevant patient has been inputted from the patient information input column 356f through the input section 34, the inputted patient information is written into the supplementary information of the extracted image data and is sent to the server 7 through the communication section 36. Then a writing request to the image DB 70 is made. After the image data has been written into the image DB 70, the termination button 356j of the captured image list display screen 356 is pressed by the input section 34, whereby the termination of medical examination is instructed. Then the image data and search ID written into the image DB 70 of the server 7 are deleted from the temporary storage section 331.

Upon completion of the examination of the patient in the doctor consultation room, the doctor uses a patient medical chart made of paper to record the medical findings of the patient (name of the disease or injury diagnosed), the dosage information indicating the medication prescribed for the patient, and the information on the image capturing and examination conducted on the patient (e.g., type of the apparatus used for image capturing, the number of captured images, presence or absence of a contrast medium, region of the image, direction of shooting, type of examination, and examination ID). The patient medical chart is handed over to the person in charge at reception desk 11.

The person in charge at the reception desk 11 displays the reception-related information input screen on the reception computer 5, and inputs the reception-related information through the reception-related information input screen based on the descriptions of the paper patient medical chart. The inputted information is stored in the reception DB 50.

The person in charge at reception desk 11 displays the examination information input screen on the reception computer 5, and inputs the examination ID and examination information. In the reception computer 5, the examination information inputted from the examination information input screen is associated with the examination ID and stored in the examination information DB 51. When requesting an external testing organization to conduct a sample test, examination information is inputted when the result of the test has been received from the testing organization on a later date.

According to the aforementioned procedure, in the small-sized diagnostic system 1, the captured image obtained by capturing an image of the patient's region to be examined is associated with the patient's information, and information on date and time of image capturing, and is stored in the image DB 70 of the server 7. The reception-related information including the treatment information such as medication dosage information, and examination information is associated with the patient information and reception date (i.e., the date of medication dosage and examination), and is stored in the reception DB 50. Further, the examination information is associated with the reception-related information by the examination ID, and is stored in the examination information DB 51.

In the conventional method, when the doctor wants to get overall information on image capturing, examination, and medication received by a patient, it has been necessary to take the trouble to find a patient's paper chart on a shelf in a medical facility wherein the diagnostic records are kept on a patient's paper chart. This has been very inconvenient. In the present embodiment, the control apparatus 3 ensures that a list of the captured image, examination information, and medication dosage information obtained from the patient designated by a doctor is displayed on the medical care summary screen 351 of the display section 35, based on the captured image stored in the image DB 70 and the reception-related information stored in the reception DB 50.

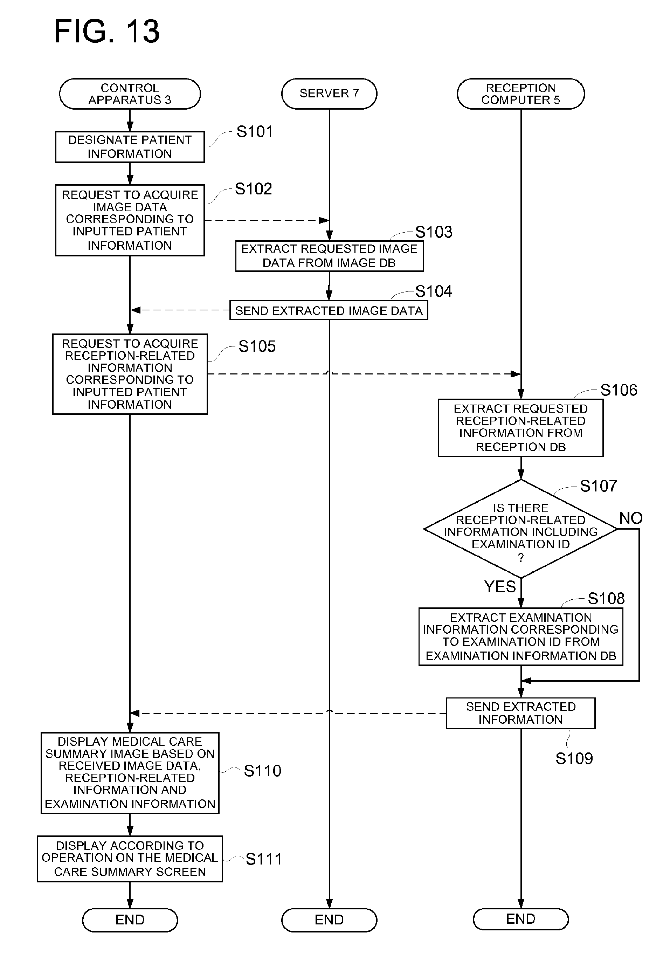

Fig. 13 shows the processing of medical care summary display implemented in the control apparatus 3 and a medical care summary display sequence carried out in response thereto in the control apparatus 3, server 7, and reception computer 5. The medical care summary display processing is implemented when the medical care summary display instructions are inputted from the input section 34. The following describes the medical care summary display sequence.

When the medical care summary display instructions have been inputted from the menu screen through the input section 34, the patient designation screen for designating the information of the patient (to be examined) as an object of the medical care summary display appears on the display section 35. Patient information designation device is implemented by the patient designation screen appearing on the display section 35 and the input section 34. When the patient information is inputted from the patient designation screen through the input section 34, the patient as the object of medical care summary display is designated (Step S101). Then, the inputted patient information is sent to the server 7 and a request to acquire the image data corresponding to this patient information is sent to the server 7 (Step S102).

In the server 7, patient information is sent from the control apparatus 3, and the request to acquire the image data corresponding to this patient information is received. Then the image data corresponding to the patient information having been received is extracted from the image DB 70 (Step S103), and the extracted image data together with the supplementary information thereof is sent to the control apparatus 3 (Step S104).

In the control apparatus 3, the image data corresponding to the patient information having been designated is acquired together with the supplementary information. Then, the patient information designated in Step S101 is sent to the reception computer 5, and a request to acquire the reception-related information corresponding to this patient is sent (Step S105).

In the reception computer 5, the patient information is received from the control apparatus 3 and a request is received to acquire the reception-related information corresponding to the patient information. Then, the reception-related information corresponding to the patient information having been received is extracted from the reception DB 50 (Step S106). When the examination ID is included in the extracted reception-related information (Yes in Step S107), the examination information identified by the examination ID is extracted from the examination information DB 51 (Step S108). The extracted reception-related information and examination information are sent to the control apparatus 3 (Step S109).

In the control apparatus 3, when the image data, the reception-related information, and the examination information corresponding to the patient information of the specified patient are received, processing of Step S110 and Step S111 is performed, and a display device is implemented. To be more specific, medical care summary screen data is generated based on the patient information from the image data, reception-related information, and examination information having been received. The medical care summary screen 351 (Fig. 5 and others) is displayed on the display section 35. Display in conformity to the functions associated with various icons and operation buttons is given in response to the operation of various icons and operation buttons displayed on the medical care summary screen 351 through the input section 34 (Step S111).

In Step S110, an medical care summary generation program is read out and the medical care summary screen data is generated in the RAM 32 according to the medical care summary generation program. Based on the medical care summary screen data, the medical care summary screen 351 appears. For example, the medical care summary screen data storage area is created in the RAM 32. Then, the patient information on the patient as the object of medical care summary display and the time period preset as a default such as the time axis up to this date from "N" months ago (wherein "N" indicates a positive integer) is written. Then an object icon (thumbnail image) representing the simplified form of the contents of the image data is generated for each piece of the image data received from the server 7 wherein the time and date of image capturing included in the supplementary information (UID) thereof are within the time period represented by the time axis. The object icons are classified according to modality (e.g., CR, US (ultrasonic diagnostic apparatus) and endoscope apparatus). The object icons A1 of the image data captured by the same modality are associated with the time axis and are arranged across the screen in the horizontal direction. Then, an object icon A2 representing the simplified form of the contents of examination information is generated for each piece of examination information received from the reception computer 5 wherein the examination date (the date of the "reception date" item of the reception-related information associated with the examination ID) is within the time period represented by the time axis. The icons are classified according to the type of examination (e.g., electrocardiogram, blood examination, physiologic examination (urine test, feces test and tissue examination)). The object icons A2 by the same examination type are associated with time axis and are arranged across the screen in the horizontal direction. Then, an object icon A3 representing the simplified form of the contents of medication dosage information is generated for each piece of the medication dosage information included in the reception-related information received from the reception computer 5 wherein the date of medication dosage (the date of the "reception date" item of the reception-related information) is within the time period represented by the aforementioned time axis. The object icon A3 is associated with the time axis, and is arranged across the screen in the horizontal direction. The object icons A1 through A3 are associated with the function of showing the details of the image data, examination information or medication dosage data each of which is a basis of the object icons. The "name of disease and injury" of the reception-related information received from the reception computer 5 wherein the date of reception is within the time period of the aforementioned time axis is associated with the time axis and is displayed across the screen in the horizontal direction. Further, the operation buttons and icons associated with various functions are generated and are placed at predetermined positions. After that, necessary displays are written. The medical care summary screen 351 appears on the display section 35, based on the medical care screen data generated in the aforementioned procedure (e.g., Fig. 6).

The object icon A2 is a simplified representation of the contents of the examination information. For example, it is also possible to make such arrangements that the aforementioned examination information input screen is provided with an item for inputting a normal or abnormal examination result, an item for inputting an abnormal result if there is any abnormal result, an item for inputting a normal or abnormal result in the previous examination if there is any abnormal result, and an item for inputting the time when an abnormal result has occurred. They can be inputted according to each item. If the examination information is stored according to these items in the examination information DB 51, the contents of the major items previously set out of these items can be displayed on the object icon A2. Further, the items to be represented by the object icon A2 can be set from the input section 34.

For the object icon A3, the "dosage" items of the reception DB 50 are classified into subdivided items, and the name of the medication to be administered, the dose per day, and the period of dosage can easily be stored. This arrangement allows the medical care summary screen 351 to provide the doctor with easy-to-see information.

Fig. 14(a) shows an example of the image comparison screen 354. On the image comparison screen 354, the reference image obtained by image capturing at the reference time specified by the arrow icon 351g in the medical care summary screen 351 and the captured image corresponding to the object icon A1 specified in the medical care summary screen 351 are displayed side by side in the comparative screen display area 354a. This arrangement allows the doctor to make easy comparisons between the reference image and the image of a desired time. Thus, the doctor has easy access to the information on how the patient has recovered from a desired time. The image for comparison with the reference image can be changed by pressing the arrow button 354c or 354d indicated on the lower portion of the captured image, or by selection of the thumbnail images displayed according to the order of image capturing. The same operation buttons as those displayed on the captured image display screen 352 of Fig. 7 and "image format" button B31 are displayed on the image comparison screen 354. When this "image format" button B31 has been pressed on the input section 34, the area for setting the number of the images to be compared with the reference image pops up, and the comparison images in the number set from this area can be displayed in the comparative screen display area 354a. For example, when the number of the comparison images is set at three, the image comparison screen 354 of Fig. 14(b) appears. In the comparative screen display area 354a, the comparison images and reference images are arranged in the order of capturing.

On the medical care summary screen 351, after the tack-pin icon 351h of Fig. 5 has been pressed on the input section 34, the object icon A1 of the image is selected. Then, the image corresponding to the object icon A1 having been selected can be set as the comparison candidate image. On the image comparison screen 354, only the thumbnail images of the comparison candidate images are displayed in the image capturing order display area 354b in the order of capturing without excessive thumbnail images being displayed. This arrangement allows easier selection of the image to be displayed side by side with the reference image in the comparative screen display area 354a.

When any one of the object icons A2 displayed in the medical care summary display area 351c of the medical care summary screen 351 has been designated from the input section 34 representing an icon designation device, the display of the display section 35 is switched to the examination information detail screen (not illustrated) displaying in detail the examination information represented by the designated icon. Similarly, in the case of the captured image display screen 352, examination information of the reference time is displayed on the examination information detail screen together with the detailed information of the examination information represented by the designated object icon A2. This arrangement allows the doctor to easily make comparisons between examination results at the reference time and that at a desired time.

When any one of the object icons A3 appearing in the medical care summary display area 351c of the medical care summary screen 351 has been designated from the input section 34 representing an icon designation device, the display of display section 35 is changed over to the medication information detail screen (not illustrated) showing in detail the medication dosage information represented by the designation object icon. Similarly, in the case of the captured image display screen 352, together with the detailed information of the medication dosage information indicated by the designated object icon, the medication dosage information for the reference time period is indicated on the medication information detail screen. This arrangement allows the doctor to easily make comparisons between the medication results for the reference time period and that for a desired time period.

The object icon A2 for the examination information which is not yet informed by the doctor to the patient is displayed on the medical care summary screen 351, with the object icon A2 preferably including the identification mark that shows that the results of this examination have not yet been informed by the doctor to the patient. For example, when the object icon A2 has been designated by the input section 34, the details of the examination information can be displayed on the display screen of the display section 35. The object icon A2 that has not been designated by the input section 34 is provided with an identification mark. After that, when the detailed information of the examination information corresponding to the object icon A2, to which designation has been given from the input section 34 has been displayed, information to the patient is regarded as having been made, and the identification mark for this object icon A2 is deleted. To show whether or not the object icon A2 of examination information has been designated from the input section 34, when the object icon A2 is designated from the input section 34, the examination ID of the examination information corresponding to the designated object icon A2 and the information showing that an explanation has been completed are sent to the reception computer 5 from the control apparatus 3. They are associated with the examination information identified by the examination ID of the examination information DB 51, and are stored. When the medical care summary screen 351 is displayed, the aforementioned identification mark can be assigned based on the examination information not corresponding to the information showing the completion of an explanation.

As described above, in the control apparatus 3, the image data corresponding to the patient information designated by the doctor and the supplementary data thereof, are acquired from the image DB 70 of the server 7, and the reception-related information including the examination information and the treatment information corresponding to the patient information is acquired from the reception computer 5. Based on the acquired captured image, examination information, and treatment information, the medical care summary screen 351 is displayed to indicate a list of the captured images, examination information, and treatment information of the patient designated by the doctor, which are arranged in a list in chronological order.

Accordingly, the doctor having a relationship with the patient which are often found in small-sized facilities (intimate relationships with the patients as a family doctor) has easy access to all of the captured images, examination information, and treatment information on the relevant patient, by the simple input operation of designating the patient, without necessity of operation to input comments of medical findings as in the case of the electronic patient medical chart. On the medical care summary screen 351, the captured image examination information and treatment information are displayed being associated with the time axis in chronological order. This arrangement allows the doctor to gain easy access to the recovery progress of the patient in chronological order. When the doctor wants to get the details of the captured image, examination information, and treatment information, the details can be displayed by merely using the input section 34 to designate any one of the object icons A1 through A3 displayed on the medical care summary screen 351. This ensures easy and accurate diagnosis through easy input operation. Even in an existing small-sized facility using a patient medical chart made of paper, if provided with an image generating apparatus 2, the captured image associated with the patient information is stored in the database or the like, and the medication dosage information and examination information associated with the patient information for calculation of the insurance points for the patient are inputted into the computer and stored therein. Thus, this arrangement realizes a small-sized diagnostic system of excellent usability characterized by easy search access to the captured image, examination information, and treatment information of a patient, by means of small modifications to the existing facility.

It should be noted that the description of the aforementioned embodiment is a preferred example of the small-sized diagnostic system 1 of the present invention without being restricted thereto.

For example, the aforementioned embodiment has been described with reference to the case of displaying a list of the captured image, examination information, and treatment information on the medical care summary screen 351. However, it is not always necessary to display all of them. It is also possible to arrange such a configuration so that the information to be displayed on the medical care summary screen 351 through the input section 34 can be set. For example, it is also possible to ensure that the captured image and examination information can be displayed on the medical care summary screen 351, or that the captured image and treatment information are displayed on the medical care summary screen 351. It is also possible to ensure that the modality of the captured image to be displayed on the medical care summary screen 351 can be set.

Instead of the image DB 38 of the control apparatus 3, the image DB 70 of the server 7 can be used as a storage device. Instead of the image DB 38, the image DB 70 can be used as an image storage device.

It goes without saying that the present invention can be modified as seen fit, without being restricted to the aforementioned embodiments.

## Claims

1. A medical imaging system in which an image generating apparatus for capturing an image of a region of a patient to be examined so as to generate image data is connected with a medical image controlling apparatus which associates the image data generated by the image generating apparatus with patient information so that data can be sent and received between the image generating apparatus and the medical image controlling apparatus, the medical image controlling apparatus comprising:
a patient list acquiring device for acquiring a patient information list of the patient to be examined;
a patient designating device for designating the patient information for the patient to be examined, from the acquired patient information list;
a control mode selecting device for selecting a control mode when the image data is received from the image generating apparatus; and
a control device having a first control mode wherein, when image data is received from the image generating apparatus, the received image data is automatically associated with the patient information designated by the patient designating device, and is stored in a storage device, and a second control mode wherein the received image data is stored in a temporary storing device without being associated with the patient information, wherein the received image data is subjected to storage control in the first or the second control mode based on the selection by the control mode selecting device.

2. The medical imaging system of claim 1,
wherein the image generating apparatus is installed in an radiographing room and the medical image controlling apparatus is installed in a doctor consultation room.

3. The medical imaging system of claim 1 or 2,
wherein the medical image controlling apparatus further comprising,
a notifying device for notifying of reception of the image data from the image generating apparatus.

4. The medical imaging system of claim 3,
wherein the notifying device notifies of the reception every time the image data is received from the image generating apparatus.

5. The medical imaging system of any one of claims 1 - 4,
wherein the medical image controlling apparatus is connected with a plurality of the image generating apparatuses.

6. The medical imaging system of claim 5,
wherein the control mode selecting device is capable of selecting the control mode in response to each of the connected image generating apparatuses.

7. The medical imaging system of any one of claims 1 - 6,
wherein the medical image controlling apparatus has further a third control mode so that when the image data is received from the image generating apparatus, the received image data is associated with patient information different from the patient information designated by the patient designating device, and
wherein when the third control mode is selected by the control mode selecting device, the control device displays the patient information list acquired by the patient list acquiring device on a display device with patient information being selectable, and associates the patient information selected from the displayed patient information list with the received image data and the received image data is stored in the storage device.

8. The medical imaging system of any one of claims 1 - 7,
wherein the medical image controlling apparatus is connected with an examination apparatus for acquiring patient examination data so that data can be sent and received therebetween,
wherein the control mode selecting device is capable of selecting the control mode when the examination data is received from the examination apparatus, and
wherein when the examination data is received from the examination apparatus, the control device automatically associates the received examination data with the patient information designated by the patient designating device based on the selection of the control mode selecting device, and stores the examination data in an examination information storing device, or stores the received examination data into a temporary storing device, without the examination data being associated with the patient information.

9. The medical imaging system of any one of claims 1 - 7 further comprising:
a reception device for registering information for separating patients individually and information on a reception date, for reception of the patient;
a patient information input device for inputting the patient information by associating the patient information with the information for separating patients individually and the information on the reception date, which have been registered by the reception device;
an image storing device for storing the image data generated by the image generating apparatus, the patient information on the patient whose image to be captured, and information on a date of capturing the image data, which have been associated with one another;
a reception-related information input device for inputting reception-related information including at least one of computerized examination information and treatment information corresponding to the information for separating patients individually and the information on the reception date registered by the reception device; and
a reception information storing device for storing the patient information and reception-related information which have been inputted and associated with each other, based on the information for separating patients individually and the information on the reception date;
wherein the medical image controlling apparatus acquires the image data and the information on the date of capturing the image data which have been associated with the patient information for patient to be examined and stored in the image storing device, and acquires at least one of the examination information and the treatment information together with the information on the reception date, which have been associated with the patient information and stored in the reception information storing device, and
wherein the medical image controlling apparatus comprises, a display device which displays the acquired image data, at least one of the examination information and the treatment information , together with the patient information in a list in chronological order on a screen by associating the acquired image data with at least one of the examination information and the treatment information.

10. The medical imaging system of claim 9,
wherein the reception-related information input device comprises the patient information input device.

11. The medical imaging system of claim 9 or 10 further comprising,
a plurality of types of the image generating apparatuses,
wherein the image storing device stores the image data generated by the image generating apparatuses, the patient information on the patient whose image to be captured, the information on the date of capturing the image data, and information on a type of the image generating apparatus, which have been associated with one another, and
wherein the display device of the medical image controlling apparatus displays the image data in a list for each type of the image generating apparatus.

12. The medical imaging system of any one of claims 9 - 11,
wherein a screen in which the image data, at least one of the examination information and the treatment information are displayed together with the patient information in a list in chronological order is configured so as to be changed to a screen for displaying a detail of each captured image having been displayed on the screen.
